# EUROPEAN PATENT SPECIFICATION

(11) **EP 3 933 036 B1**
(45) Date of publication and mention of the grant of the patent: **17.12.2025**
(21) Application number: 20183693.9
(22) Date of filing: 02.07.2020
(51) Int. Cl.: C12N 9/02, C12P 33/06

(54) **PROCESS FOR 7-BETA-HYDROXYLATION OF BILE ACID DERIVATIVES**
VERFAHREN ZUR 7-BETA-HYDROXYLIERUNG VON GALLENSÄUREDERIVATEN
PROCÉDÉ D'HYDROXYLATION 7-BÊTA DE DÉRIVÉS D'ACIDE BILIAIRE

(43) Date of publication of application: 05.01.2022
(73) Proprietor: MICROMUN Institut für Mikrobiologische Forschung GmbH, 17390 Murchin (DE)
(72) Inventor: Bornscheuer, Uwe, 17489 Greifswald (DE); Grobe, Sascha, 17489 Greifswald (DE); Hannevik, Emil, 75267 Uppsala (SE); Bayer, Thomas, 8020 Graz (AT); Badenhorst, Christoffel, 17489 Greifswald (DE); Brundiek, Henrike, 49525 Lenggerich (DE); Großjohann, Beatrice, 17498 Mesekenhagen (DE)
(74) Representative: Altmann Stößel Dick Patentanwälte PartG mbB

(56) References cited:
- WO-A1-2016/016213
- WO-A1-2020/076819
- CN-A- 109 055 473
- US-A1- 2001 034 046
- KOLLEROV V V ET AL: "Hydroxylation of lithocholic acid by selected actinobacteria and filamentous fungi", STEROIDS, ELSEVIER SCIENCE PUBLISHERS, NEW YORK, NY, US, vol. 78, no. 3, 18 January 2013 (2013-01-18), pages 370 - 378, XP028981316, ISSN: 0039-128X, DOI: 10.1016/J.STEROIDS.2012.12.010
- GROBE SASCHA ET AL: "Highly selective bile acid hydroxylation by the multifunctional bacterial P450 monooxygenase CYP107D1 (OleP)", BIOTECHNOLOGY LETTERS, KLUWER ACADEMIC PUBLISHERS, DORDRECHT, vol. 42, no. 5, 23 January 2020 (2020-01-23), pages 819 - 824, XP037078322, ISSN: 0141-5492, [retrieved on 20200123], DOI: 10.1007/S10529-020-02813-4
- FABIO TONIN ET AL: "Latest development in the synthesis of ursodeoxycholic acid (UDCA): a critical review", BEILSTEIN JOURNAL OF ORGANIC CHEMISTRY, vol. 14, 5 February 2018 (2018-02-05), pages 470 - 483, XP055657131, DOI: 10.3762/bjoc.14.33
- SASCHA GROBE ET AL: "Engineering Regioselectivity of a P450 Monooxygenase Enables the Synthesis of Ursodeoxycholic Acid via 7[beta]-Hydroxylation of Lithocholic Acid", ANGEWANDTE CHEMIE INTERNATIONAL EDITION, vol. 60, no. 2, 11 January 2021 (2021-01-11), pages 753 - 757, XP055771624, ISSN: 1433-7851, DOI: 10.1002/anie.202012675

## Description

A first aspect of the invention relates to a process for 7-beta-hydroxylation of bile acid derivatives comprising: (a) providing a bile acid derivative according to general formula (I); (b) contacting the bile acid derivative according to general formula (I) with at least one cytochrome P450 monooxygenase analog, wherein the cytochrome P450 monooxygenase analog comprises an amino acid sequence according to SEQ ID NO: 1 with at least two mutations compared to the parent cytochrome P450 monooxygenase; thereby obtaining a bile acid derivative according to general formula (II) as a product, which has a beta-hydroxyl group in position 7. In a second aspect, the invention is related to a cytochrome P450 monooxygenase analog, wherein the parent cytochrome P450 monooxygenase of the cytochrome P450 monooxygenase analog is cytochrome P450 monooxygenase CYP107D1 (OleP) from *Streptomyces antibioticus* according to SEQ ID NO: 1, wherein the cytochrome P450 monooxygenase analog comprises at least two mutations compared to the parent cytochrome P450 monooxygenase at positions selected from the group consisting of 84, 240, and 291. A third aspect of the invention is related to a polynucleotide encoding the cytochrome P450 monooxygenase analog of the third aspect. In a fourth aspect, the invention is related to a vector comprising the polynucleotide encoding the cytochrome P450 monooxygenase analog of the second aspect. A fifth aspect of the invention relates to a host cell comprising the cytochrome P450 monooxygenase analog of the second aspect and/or the polynucleotide of the third aspect, and/or the vector of the fourth aspect.

Ursodeoxycholic acid (UDCA) is a valuable bile acid frequently prescribed for the treatment of gallstones and other cholestatic diseases. UDCA can be used to solubilize cholesterol gallstones with fewer side effects than chenodeoxycholic acid (CDCA). UDCA is applied in the therapy of liver diseases like primary biliary cholangitis and cystic fibrosis and has antiinflammatory properties.

As starting materials for the synthesis of UDCA, cholic acid (CA) or CDCA are frequently used. The synthesis route starting from CA via 12-oxo CDCA, COCA and 7-oxo LCA forms CDCA within four steps including a Wolff-Kishner reduction followed by epimerization at position 7 to form UDCA. The yields do not exceed 30%. To overcome these limitations, efforts have been made in the development of shorter synthesis routes using chemoenzymatic approaches focusing on the epimerization of CDCA to UDCA. Alternatively, a number of microbial biotransformations have been reported to form UDCA from litocholic acid (LCA). For example the fungi *Fusarium equiseti* converts LCA to a number of products, including UDCA at 35% yield (see S Kulprecha, T Ueda, T Nihira, T Yoshida and H Taguchi, Appl. Environ. Microbiol. 1985, 1985, 338). Currently, there is no enzyme known to specifically hydroxylate LCA at the 7-beta position to form UDCA. Such an enzyme would be a valuable tool for direct conversion of LCA to UDCA without involving the complex metabolism of fungi that inanalogly produce multiple undesired side products.

A major challenge in the enzymatic synthesis of UDCA from LCA is the hydrophobicity and extremely low solubility of LCA in water, compared to the more widely used CA or CDCA. However, the great advantage of using LCA rather than CA or CDCA as starting material is its inexpensive price and its abundant availability as waste product from meat production as this is one of the major bile compounds in farmed animals like cattle and pigs compared to the aforementioned bile acids. Until now no value-added process is known utilizing LCA originating from these resources, which makes it a desirable starting material for the synthesis of UDCA.

Cytochrome P450 proteins are heme-containing enzymes capable of stereo- and regioselective hydroxylation reactions of a wide variety of substrates, using molecular oxygen as oxidant. Due to its potential for late-stage hydroxylation of industrially relevant precursor compounds, this enzyme class has been intensively investigated. Protein engineering is often employed to produce P450 monooxygenases that meet the requirements of excellent regio- and stereoselectivity for the desired applications.

The Cytochrome P450 +monooxygenase CYP107D1 (OleP, wild type) originally described for an epoxidation in the oleandomycin biosynthesis pathway was found to also accept 12 membered macrolactones as substrates. OleP can also hydroxylate testosterone at the 7-beta position. Unfortunately, it mono-hydroxylates bile acids like LCA only at the 6-beta position forming MDCA (see S. Grobe, A. Wszo 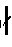 ek, H. Brundiek, M. Fekete, U. T. Bornscheuer, *Biotechnol. Lett.* **2020,** 819) as shown below in Scheme 1:

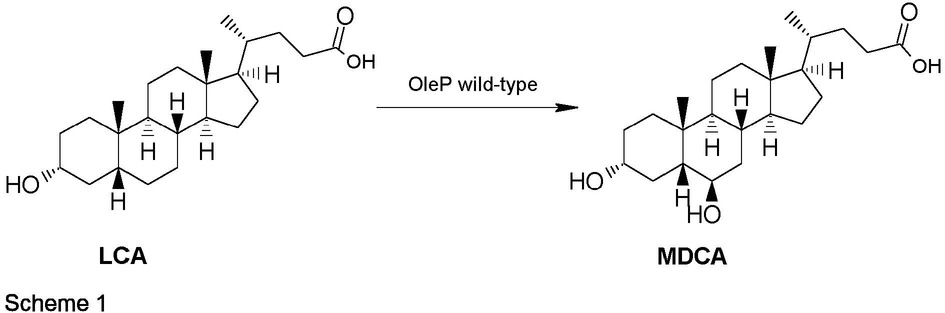

In view of the growing demand for UDCA, there is still a need to provide UDCA in greater amounts and based on a synthesis, which produces no or only minor amounts of side products.

The objective technical problem underlying the present invention was thus the provision of a process for providing bile acids, which are beta-hydroxylated in position 7 such as UDCA, wherein the method should be able to provide the desired 7-beta-hydroxylated bile acid derivative free of side products.

The object was solved by a process for 7-beta-hydroxylation of bile acid derivatives comprising:
(a) providing a bile acid derivative according to general formula (I) 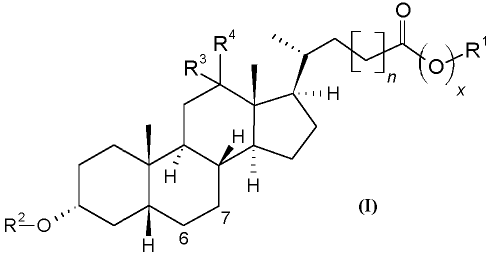 wherein
   - R¹: is selected from the group consisting of hydrogen atom, C1 to C30 alkyl group, C1 to C30 alkenyl group, C1 to C30 alkinyl group, C5 to C12 cycloalky group, C5 to C12 aryl group; and NR⁵R⁶ group, wherein R⁵ and R⁶ are independently selected from hydrogen atom, C1 to C10 alkyl group, C5 to C12 aryl group, - C(=O)-C1 to C10 alkyl group, -C(=O)-C5 to C12 aryl group and -NH-C1 to C5 alkyl-S(=O)₂-OH group;
   - R²: is selected from the group consisting of hydrogen atom, -C(=O)-C1 to C30 alkyl group, -C(=O)-C5 to C12 aryl group -S(=O)₂-C1 to C30 alkyl group, - S(=O)₂-C5 to C12 aryl group, -S(=O)₂-OH group, salt of a -S(=O)₂-OH group, - P(=O)-OH-R⁷ group, salt of -P(=O)-OH-R⁷ group, wherein R⁷ is a hydrogen atom or a C1 to C5 alkyl group, -P(=O)(OH)₂ group, and a salt of -P(=O)(OH)₂ group;
   - R³, R⁴: are independently selected from hydrogen atom or -OR², wherein R² has the same meaning as above; or R³ and R⁴ together form =O;
   - n: is zero or an integer in the range of from 1 to 5;
   - x: is zero or 1;
(b) contacting the bile acid derivative according to general formula (I) with at least one cytochrome P450 monooxygenase analog, wherein the cytochrome P450 monooxygenase analog comprises an amino acid sequence according to SEQ ID NO: 1 with at least two mutations compared to the parent cytochrome P450 monooxygenase at positions selected from the group consisting of 84, 240, and 291 based on SEQ ID NO:1, wherein the parent cytochrome P450 monooxygenase of the cytochrome P450 monooxygenase analog is cytochrome P450 monooxygenase CYP107D1 (OleP) from *Streptomyces antibioticus* according to SEQ ID NO: 1;

thereby obtaining a bile acid derivative according to general formula (II) as a product, which has a beta-hydroxyl group in position 7, 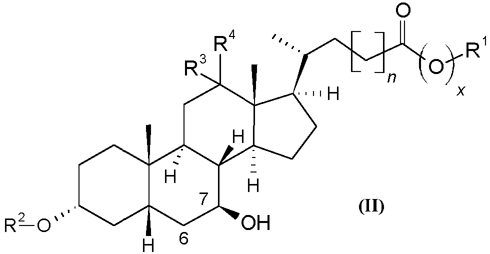
wherein R¹, R², R³, R⁴ and the indices n and x have the same meaning as for the bile acid derivative according to general formula (I).

The terms "salt of a -S(=O)₂-OH group", "salt of -P(=O)-OH-R⁷ group", and "salt of - P(=O)(OH)₂ group" are referring to pharmaceutically acceptable salts of these groups. Pharmaceutically acceptable salts are known to the person skilled in the art and are described, for example, in Berge et al. "Pharmaceutical Salts", J. Pharm. Sci. 66: 1-19 (1977). "Pharmaceutically acceptable salt" means a non-toxic, organic or inorganic acid addition salt of a bile acid derivative according to general formula (I) having a -S(=O)₂-OH group , a -P(=O)-OH-R⁷ group, or a -P(=O)(OH)₂ group, the compound of the general formula (I) being present in the acid addition salt in cationic form. The pharmaceutically acceptable salt has a neutral charge overall. The anion of the pharmaceutically acceptable salt is preferably selected from the group consisting of sulfate, pyrosulfate, bisulfate, sulfite, bisulfite, phosphate, monohydrogen phosphate, dihydrogen phosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylene sulfonate, phenylacetate, phenylpropionate, phenylbutyrate, citrate, lactate, beta-hydroxybutyrate, glycolate, tartrate, methanesulfonate, propanesulfonate, naphthalene-1-sulfonate, naphthalenate, naphthalenate, naphthalenate, naphthalenate, naphthalenate, mucate, aspartate, benzenesulfonate, butyrate, camphorate, camphorsulfonate, cyclopentane propionate, digluconate, dodecyl sulfate, ethanesulfonate, glucoheptanoate, glycerophosphate, hemisulfate, hexanoate, 2-hydroxyethane sulfonate, 2-naphthalene sulfonate, nicotinate, nitrate, palmitate, pectinate, persulfate, picrate, pivalate, salicylate, thiocyanate, tosylate, arginate, undecanoate and mixtures of two or more of these anions.

In one embodiment, the bile acid derivative according to general formula (I) having a -S(=O)₂-OH group , a -P(=O)-OH-R⁷ group, or a -P(=O)(OH)₂ group is used as an acid addition salt in the form of the chloride, sulfate, mesylate, besylate, tosylate or the mono-, di- or tricarboxylic acid salt, the mono-, di- or tricarboxylic acid is preferably selected from the group consisting of fumaric acid, malonic acid, malic acid, succinic acid, lactic acid, glycolic acid, maleic acid, citric acid, aspartic acid and mandelic acid. The acid addition salts comprise also mixtures of two or more thereof.

The bile acid derivative according to general formula (I) is used alone or as a mixture of two or more bile acid derivatives according to general formula (I).

According to a preferred embodiment of the process, R' is selected from the group consisting of hydrogen atom, C1 to C30 alkyl group, C1 to C30 alkenyl group, C1 to C30 alkinyl group, C5 to C12 cycloalky group and C5 to C12 aryl group, wherein R¹ is preferably a hydrogen atom or a C1 to C30 alkyl group, more preferably a hydrogen atom.

According to a further preferred embodiment of the process, R² is a hydrogen atom or a - C(=O)-C1- to C30 alkyl group, preferably a hydrogen atom.

According to a further preferred embodiment of the process,
- R¹: is selected from the group consisting of hydrogen atom, C1 to C30 alkyl group, C1 to C30 alkenyl group, C1 to C30 alkinyl group, C5 to C12 cycloalky group and C5 to C12 aryl group, wherein R¹ is preferably a hydrogen atom or a C1 to C30 alkyl group, more preferably a hydrogen atom;
- R²: is a hydrogen atom or a -C(=O)-C1- to C30 alkyl group, preferably a hydrogen atom;
and R³, R⁴ have the meaning as indicated above. Preferably, the index n is zero. The index x is preferably 1.

According to a preferred embodiment of the process,
- R¹: is selected from the group consisting of hydrogen atom, C1 to C30 alkyl group, C1 to C30 alkenyl group, C1 to C30 alkinyl group, C5 to C12 cycloalky group and C5 to C12 aryl group, wherein R¹ is preferably a hydrogen atom or a C1 to C30 alkyl group, more preferably a hydrogen atom;
- R²: is a hydrogen atom or a -C(=O)-C1- to C30 alkyl group, preferably a hydrogen atom;
- n: is zero;
- x: is 1; and
- R³, R⁴: have the meaning as in claim 1.

According to a preferred embodiment of the process, R³, R⁴ are each a hydrogen atom or R³ and R⁴ together form =O.

According to a further preferred embodiment of the process,
- R¹: is selected from the group consisting of hydrogen atom, C1 to C30 alkyl group, C1 to C30 alkenyl group, C1 to C30 alkinyl group, C5 to C12 cycloalky group and C5 to C12 aryl group, wherein R¹ is preferably a hydrogen atom or a C1 to C30 alkyl group, more preferably a hydrogen atom;
- R²: is a hydrogen atom or a -C(=O)-C1- to C30 alkyl group, preferably a hydrogen atom;
- n: is zero;
- x: is zero; and
- R³, R⁴: are each a hydrogen atom or R³ and R⁴ together form =O.

According to a further preferred embodiment of the process, the bile acid derivative according to general formula (II) having a beta-hydroxyl group in position 7 is obtained with a bile acid derivative according to general formula (III) as a further product having a beta-hydroxyl group in position 6 (instead of position 7), 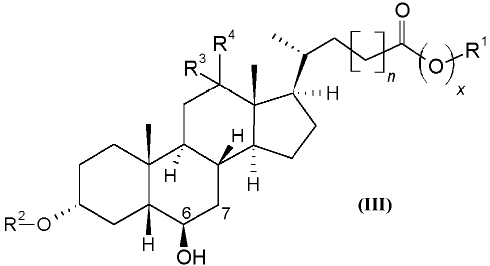
wherein R¹, R², R³, R⁴, R⁵ and the indices n and x have the same meaning as for the bile acid derivative according to general formula (I) in any one of claims 1 to 9,
wherein the molar ratio (II):(III) is in the range of from 1:10 to 100:0.001, preferably in the range of from 4:10 to 100:0.001, more preferred in the range of from 1:1 to 100:0.001.

According to a further preferred embodiment of the process for 7-beta-hydroxylation of bile acid derivatives as described above, the process comprises:
(a) providing a bile acid derivative according to general formula (la), 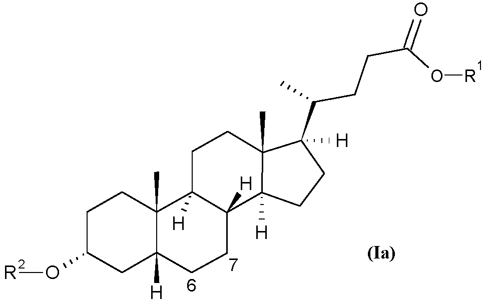 wherein
   - R¹: is selected from the group consisting of hydrogen atom, C1 to C30 alkyl group, C1 to C30 alkenyl group, C1 to C30 alkinyl group, C5 to C12 cycloalky group and C5 to C12 aryl group, preferably a hydrogen atom or a C1 to C30 alkyl group, more prefered a hydrogen atom;
   - R²: is a hydrogen atom or a -C(=O)-C1- to C30 alkyl group, preferably a hydrogen atom;
(b) contacting the bile acid derivative according to general formula (Ia) with at least one cytochrome P450 monooxygenase analog, wherein the cytochrome P450 monooxygenase analog comprises at least two mutations compared to the parent cytochrome P450 monooxygenase;

thereby obtaining a bile acid derivative according to general formula (IIa) as a product, which has a beta-hydroxyl group in position 7, 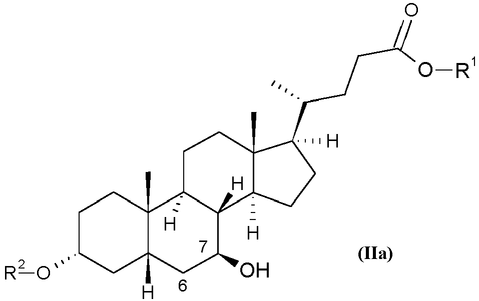
wherein R¹ and R² have the same meaning as for the bile acid derivative according to general formula (la) above.

According to a further preferred embodiment of the process the bile acid derivative according to general formula (IIa) having a beta-hydroxyl group in position 7 is obtained with a bile acid derivative according to general formula (Illa) as a further product having a beta-hydroxyl group in position 6 (instead of position 7), 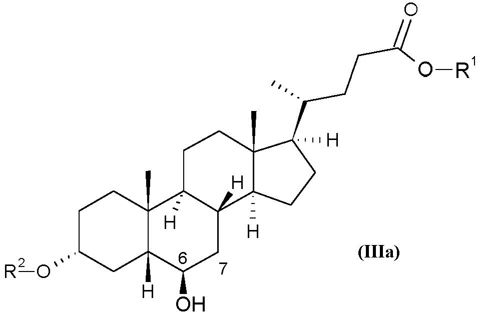 wherein R¹ and R² have the same meaning as for the bile acid derivative according to general formula (la) in claim 10, wherein the molar ratio (IIa) : (IIIa) is in the range of from 1:10 to 100:0.001, preferably in the range of from 4:10 to 100:0.001, more preferred in the range of from 1:1 to 100:0.001.

According to a further preferred embodiment of the process, the bile acid derivative according to general formula (I) or (la) provided in (a) is lithocholic acid (LCA, R¹ = hydrogen atom, R², R³, R⁴ = hydrogen atom, n = 1, x = 1) and the bile acid derivative obtained by the process having general formula (II) or (IIa) is ursodeoxycholic acid (UDCA, each of R¹, R², R³, R⁴ = hydrogen atom, n = 1, x = 1).

The bile acid derivative according to general formula (III) or (IIIa) having a beta-hydroxyl group in position 6 instead of position 7 is preferably murideoxycholic acid (MDCA, each of R¹, R², R³, R⁴ = hydrogen atom, n = 1, x = 1).

According to a further preferred embodiment of the process, the cytochrome P450 monooxygenase analog comprises an amino acid sequence according to SEQ ID NO:1 having at least two mutations compared to the parent cytochrome P450 monooxygenase at positions selected from the group consisting of 84, 240, and 291 based on SEQ ID NO:1. The gene of cytochrome P450 monooxygenase CYP107D1 (OleP) from *Streptomyces antibioticus* according to SEQ ID NO: 1 is registered in the GenBank under the accessionnumber L37200 (version L37200.1). The entry also comprises the amino acid sequence of cytochrome P450 monooxygenase CYP107D1 (OleP) from *Streptomyces antibioticus according* to SEQ ID NO: 1.

According to a further preferred embodiment of the process the at least two mutations comprised in the cytochrome P450 monooxygenase analog are that:
- position 84 is substituted with a non-aromatic amino acid, preferably with a non-aromatic amino acid selected from the group consisting of cysteine (Cys, C), glutamine (Gln, Q), methionine (Met, M), and alanine (Ala, A); and/or
- position 240 is substituted with alanine (Ala, A); and/or
- position 291 is substituted with an aliphatic or acidic amino acid, preferably with an amino acid selected from the group consisting of alanine (Ala, A), aspartic acid (Asp, D), and glycine (Gly, G).

Preferably, the cytochrome P450 monooxygenase analog comprises at least a substitution at position 291 and at least one further substitution at position 84 and/or 241.

According to a further preferred embodiment of the process, the cytochrome P450 monooxygenase analog comprises mutations in that:
- position 291 is substituted with an aliphatic or acidic amino acid, preferably with an amino acid selected from the group consisting of alanine (Ala, A), aspartic acid (Asp, D), and glycine (Gly, G); and
- position 84 is substituted with a non-aromatic amino acid, preferably with a non-aromatic amino acid selected from the group consisting of cysteine (Cys, C), glutamine (Gln, Q), methionine (Met, M), and alanine (Ala, A); and/or
- position 240 is substituted with alanine (Ala, A).

According to a further preferred embodiment of the process, the cytochrome P450 monooxygenase analog comprises or consists of an amino acid sequence selected from the group consisting of SEQ ID NO: 2 (F84Q/V291A), SEQ ID NO: 3 (S240A/V291A), SEQ ID NO: 4 (F84A/V291A), SEQ ID NO: 5 (F84C/S240A/V291D), SEQ ID NO: 6 (F84C/S240A/V291A), SEQ ID NO: 7 (F84C/S240A/V291G), SEQ ID NO: 8 (F84Q/S240A/V291A), SEQ ID NO: 9 (F84M/S240A/V291A), SEQ ID NO: 10 (F84Q/S240A/V291D), SEQ ID NO: 11 (F84A/S240A/V291A) and SEQ ID NO: 12 (F84Q/S240A/V291G).

Preferably, the cytochrome P450 monooxygenase analog comprises or consists of an amino acid sequence according to SEQ ID NO: 12 (F84Q/S240A/V291G).

According to a preferred embodiment, the cytochrome P450 monooxygenase analog comprises, in addition to the mutations compared to the amino acid sequence according to SEQ ID NO: 1, one or more further amino acid(s) prior to the first amino acid of SEQ ID NO: 1 and/or one or more further amino acid(s) after the last amino acid of SEQ ID NO: 1, preferably at least one or more further amino acid(s) prior to the first amino acid of SEQ ID NO: 1. More preferred, the cytochrome P450 monooxygenase analog comprises an amino acid sequence MGSSHHHHHH SSGLVPRGSH prior to the first amino acid. In embodiments where such an amino acid sequence MGSSHHHHHH SSGLVPRGSH prior to the first amino acid is present, the complete sequence comprises 427 amino acids and the at least two mutations are at positions selected from the group consisting of 104, 260, and 311.

These cytochrome P450 monooxygenase analogs having an amino acid sequence MGSSHHHHHH SSGLVPRGSH prior to the first amino acid are selected from the group consisting of SEQ ID NO: 13 (F104Q/V311A), SEQ ID NO: 14 (S260A/V311A), SEQ ID NO: 15 (F104A/V311A), SEQ ID NO: 16 (F104C/S260A/V/311D), SEQ ID NO: 17 (F104C/S260A/V311A), SEQ ID NO: 18 (F104C/S260A/V311G), SEQ ID NO: 19 (F104Q/S260A/V311A), SEQ ID NO: 20 (F104M/S260A/V311A), SEQ ID NO: 21 (F104Q/S260A/V311D), SEQ ID NO: 22 (F104A/S260A/V311A) and SEQ ID NO: 23 (F104Q/S260A/V311G). Preferably, the cytochrome P450 monooxygenase analog comprises or consists of an amino acid sequence according to SEQ ID NO: 23 (F104Q/S260AN3111G).

In one embodiment, the cytochrome P450 monooxygenase analog consists of an amino acid sequence selected from the group consisting of SEQ ID NO: 2 (F84Q/V291A), SEQ ID NO: 3 (S240A/V291A), SEQ ID NO: 4 (F84A/V291A), SEQ ID NO: 5 (F84C/S240A/V291D), SEQ ID NO: 6 (F84C/S240A/V291A), SEQ ID NO: 7 (F84C/S240A/V291G), SEQ ID NO: 8 (F84Q/S240A/V291A), SEQ ID NO: 9 (F84M/S240A/V291A), SEQ ID NO: 10 (F84Q/S240A/V291D), SEQ ID NO: 11 (F84A/S240A/V291A), SEQ ID NO: 12 (F84Q/S240A/V291G), SEQ ID NO: 13 (F104Q/V311A), SEQ ID NO: 14 (S260AN311A), SEQ ID NO: 15 (F104A/V311A), SEQ ID NO: 16 (F104C/S260A/V311D), SEQ ID NO: 17 (F104C/S260AN311A), SEQ ID NO: 18 (F104C/S260A/V311G), SEQ ID NO: 19 (F104Q/S260A/V311A), SEQ ID NO: 20 (F104M/S260A/V311A), SEQ ID NO: 21 (F104Q/S260A/V311D), SEQ ID NO: 22 (F104A/S260A/V311A) and SEQ ID NO: 23 (F104Q/S260AN311G).

According to a further preferred embodiment of the process, in step (b), a single cytochrome P450 monooxygenase analog or a mixture of two or more cytochrome P450 monooxygenase analogs is used, preferably a single cytochrome P450 monooxygenase analog or a mixture of two or more cytochrome P450 monooxygenase analogs, wherein each cytochrome P450 monooxygenase analog is preferably selected from the cytochrome P450 monooxygenase analogs defined in any one of claims 6 to 11, wherein more preferred the single cytochrome P450 monooxygenase analog or the mixture of two or more cytochrome P450 monooxygenase analogs comprises at least the cytochrome P450 monooxygenase analog as disclosed above.

Preferably, (b) is conducted in an aqueous solution, which preferably comprises at least 80 weight-%, more preferably at least 90 weight-%, more preferably at least 95 weight-%, more preferably at least 98 weight-%, water, based on the overall weight of the aqueous solution.

Preferably the aqueous solution comprises a buffer, more preferred selected from the group of tris(hydroxymethyl)aminomethane buffer (TRIS buffer), 3-(N-morpholino)propanesulfonic acid buffer (MOPS buffer), N,N-Bis(2-hydroxyethyl)-2-aminoethanesulfonic acid buffer (BES buffer), N-(tris(hydroxymethyl)methyl)glycine buffer (Tricine buffer), Carbonate buffer, N-cyclohexyl-2-aminoethanesulfonic acid buffer (CHES buffer), 4-(2-hydroxyethyl)-1-piperazineethanesulfonic acid buffer (HEPES buffer) and phosphate buffered saline (PBS), more preferably at least a PBS.

Preferably, step (b) is conducted at a pH value in the range of from 3.0 to 12, more preferred in the range of from 6.0 to 11, more preferred in the range of from 6.0 to 8.5.

Preferably, step (b) is conducted for a time period of at least one hour, more preferred for a time in the range of from 1 to 100 hours, more preferably in the range of from 5 to 76 hours, more preferably in the range of from 10 to 48 hours, more preferred in the range of from 15 to 36 hours, more preferred in the range of from 20 to 30 hours.

Preferably, step (b) is conducted at a temperature in the range of from 10 to 40°C, more preferred in the range of from 15 to 30°C, more preferred in the range of from 20 to 29 °C, more preferred at a temperature in the range of from 22 to 28°C.

Preferably, the bile acid derivative according to general formula (I) or (la) is provided in a solution comprising an organic solvent, more preferred an organic polar solvent, more preferred an organic polar solvent having an octanol/water partition coefficient (at 298 K) in the range from 0 to 25, more preferred in the range from 2 to 22, more preferred an organic solvent selected from the group consisting of ethanol (KOW 22), acetonitrile (KOW 2), DMSO (KOW 2) and mixtures of two or more of these solvents, more preferred an organic solvent comprising at least DMSO (KOW 2).

The octanol water/partition coefficients (KOW) as indicated herein are from the Dortmunder Datenbank, version: December 2019. The solution comprises the organic solvent in an amount in the range of from 0.001 to 10 weight-%, based on the overall weight of the solution. In case that the organic solvent comprises only one or more organic polar protic solvents, it is preferred that the solution comprises the one or more organic polar protic solvents in an amount in the range of from 0.001 to 2 weight-%. In case the organic solvent comprises only organic polar aprotic organic solvents, it is preferred that these one or more organic polar aprotic solvents are comprised in the solution in an amount in the range of from 0.001 to 10 weight-%, preferably from 0.001 to 5 weight-%, based on the overall weight of the solution. In case of mixtures of one or more organic polar aprotic solvents with one or more organic polar protic solvents, the solution comprises the one or more organic polar protic solvents in an amount in the range of from 0.001 to 2 weight-% and the one or more organic polar aprotic solvents in an amount in the range of from 0.001 to 8 weight-%, preferably in the range of from 0.001 to 3 weight-%.

Preferably,the at least one cytochrome P450 monooxygenase analog is provided in combination with a host cell, more preferred within a host cell, wherein the host cell is preferably selected from the group consisting of bacteria cell, yeast cell and fungi, wherein the yeast cell is preferably selected from *Saccharomyces cerevisiae* cell, *Picha pastoris* cell and *Yarrowia lipolytica* cell, the fungal cell is preferably an *Aspergillus sp.* cell, the bacteria cell is preferably selected from bacteria cell belonging to the genus of *Bacillus* or bacteria cell belonging to the genus *Escherichia,* more preferred the host cell is a bacteria cell, more preferred a bacteria cell belonging to the genus *Escherichia,* more preferred an *Escherichia coli* cell, more preferred an *Escherichia coli* cell selected from the group consisting of BL21 (DE3), BL21 (DE3) Gold, C41 (DE3) and C43(DE3), more preferred *E. coliC43* (DE3), more preferred DE3 delta Ipp normalized to OD₆₀₀ of 30. The OD₆₀₀ is the optical density of a sample measured at a wavelength of 600 nm with a spectrophotometer, preferably an UV Vis spectrometer.

In a further preferred embodiment, the process comprises the further steps:
(c) separating a supernatant from the host cells after step (b);
(d) optionally adjusting the pH value of the supernatant obtained in (c) to a pH value below 7, preferably in the range of from 1 to 6.9, more preferred from 3 to 6.9, thereby obtaining an acidified supernatant;
(e) isolating the bile acid derivative according to general formula (II) or (IIa) from the supernatant obtained in (c) and/or from the acidified supernatant obtained in (d), wherein isolation is preferably done by extraction, preferably with an organic solvent, preferably an organic polar aprotic solvent, more preferred an organic polar aprotic solvent having an octanol/water partition coefficient (at 298 K) in the range of from 0 to 15, more preferred in the range of from 1 to 12, more preferred by extraction with an organic solvent comprising at least ethyl acetate (KOW 11).

### 2nd aspect - cytochrome P450 monooxygenase analog

In a second aspect, the invention relates to a cytochrome P450 monooxygenase analog, wherein the parent cytochrome P450 monooxygenase of the cytochrome P450 monooxygenase analog is cytochrome P450 monooxygenase CYP107D1 (OleP) from *Streptomyces antibioticus* according to SEQ ID NO: 1, wherein the cytochrome P450 monooxygenase analog comprises an amino acid sequence according to SEQ ID NO: 1 having at least two mutations compared to the parent cytochrome P450 monooxygenase at positions selected from the group consisting of 84, 240, and 291 based on SEQ ID NO: 1.

Cytochrome P450 monooxygenase analogs provided herein comprise at least two substitutions relative to parent cytochrome P450 monooxygenase. The term "at least two", as used herein means two, or more than two, such as "at least three", "at least four", "at least five", etc., preferably "at least two" means two or three substitutions.

The cytochrome P450 monooxygenase analogs provided herein may comprise mutations in addition to the substitutions above. In some embodiments, the number of mutations does not exceed a certain number. In some embodiments, the cytochrome P450 monooxygenase analogs comprise less than twelve mutations (i.e. deletions, substitution, additions) relative to the parent cytochrome P450 monooxygenase. In another embodiment, the analog comprises less than eleven mutations relative to the parent cytochrome P450 monooxygenase. In another embodiment, the analog comprises less than ten mutations relative to the parent cytochrome P450 monooxygenase. In another embodiment, the analog comprises less than nine mutations relative to the parent cytochrome P450 monooxygenase. In another embodiment, the analog comprises less than eight mutations relative to the parent cytochrome P450 monooxygenase. In another embodiment, the analog comprises less than seven mutations relative to the parent cytochrome P450 monooxygenase. In another embodiment, the analog comprises less than six mutations relative to the parent cytochrome P450 monooxygenase. In another embodiment, the analog comprises less than five mutations relative to the parent cytochrome P450 monooxygenase.

The expression "parent cytochrome P450 monooxygenase" as used herein refers to naturally occurring cytochrome P450 monooxygenase, i.e. to unmutated wild-type parent cytochrome P450 monooxygenase CYP107D1 from *Streptomyces antibioticus according* to SEQ ID NO: 1.

According to a preferred embodiment of the cytochrome P450 monooxygenase analog, the at least two mutations comprised in the cytochrome P450 monooxygenase analog are that:
- position 84 is substituted with a non-aromatic amino acid, preferably with a non-aromatic amino acid selected from the group consisting of cysteine (Cys, C), glutamine (Gln, Q), methionine (Met, M), and alanine (Ala, A); and/or
- position 240 is substituted with alanine (Ala, A); and/or
- position 291 is substituted with an aliphatic or acidic amino acid, preferably with an amino acid selected from the group consisting of alanine (Ala, A), aspartic acid (Asp, D), and glycine (Gly, G).

Preferably, the cytochrome P450 monooxygenase analog comprises at least a substitution at position 291 and at least one further substitution at position 84 and/or 241.

According to a preferred embodiment, the cytochrome P450 monooxygenase analog comprises mutations in that:
- position 291 is substituted with an aliphatic or acidic amino acid, preferably with an amino acid selected from the group consisting of alanine (Ala, A), aspartic acid (Asp, D), and glycine (Gly, G); and
- position 84 is substituted with a non-aromatic amino acid, preferably with a non-aromatic amino acid selected from the group consisting of cysteine (Cys, C), glutamine (Gln, Q), methionine (Met, M), and alanine (Ala, A); and/or
- position 240 is substituted with alanine (Ala, A).

Preferably, the cytochrome P450 monooxygenase analog comprises or consists of an amino acid sequence selected from the group consisting of SEQ ID NO: 2 (F84Q/V291A), SEQ ID NO: 3 (S240A/V291A), SEQ ID NO: 4 (F84A/V291A), SEQ ID NO: 5 (F84C/S240A/V291D), SEQ ID NO: 6 (F84C/S240A/V291A), SEQ ID NO: 7 (F84C/S240A/V291G), SEQ ID NO: 8 (F84Q/S240A/V291A), SEQ ID NO: 9 (F84M/S240A/V291A), SEQ ID NO: 10 (F84Q/S240A/V291D), SEQ ID NO: 11 (F84A/S240A/V291A) and SEQ ID NO: 12 (F84Q/S240A/V291G). More preferred, the cytochrome P450 monooxygenase analog comprises or consists of an amino acid sequence according to SEQ ID NO: 12 (F84Q/S240A/V291G).

According to a preferred embodiment, the cytochrome P450 monooxygenase analog comprises, in addition to the mutations compared to the amino acid sequence according to SEQ ID NO: 1, one or more further amino acid(s) prior to the first amino acid of SEQ ID NO: 1 and/or one or more further amino acid(s) after the last amino acid of SEQ ID NO: 1, preferably at least one or more further amino acid(s) prior to the first amino acid of SEQ ID NO: 1. More preferred, the cytochrome P450 monooxygenase analog comprises an amino acid sequence MGSSHHHHHH SSGLVPRGSH prior to the first amino acid. In embodiments where such an amino acid sequence MGSSHHHHHH SSGLVPRGSH prior to the first amino acid is present, the complete sequence comprises 427 amino acids and the at least two mutations are at positions selected from the group consisting of 104, 260, and 311.

These cytochrome P450 monooxygenase analogs having an amino acid sequence MGSSHHHHHH SSGLVPRGSH prior to the first amino acid are selected from the group consisting of SEQ ID NO: 13 (F104Q/V311A), SEQ ID NO: 14 (S260A/V311A), SEQ ID NO: 15 (F104A/V311A), SEQ ID NO: 16 (F104C/S260A/V/311D), SEQ ID NO: 17 (F104C/S260A/V311A), SEQ ID NO: 18 (F104C/S260A/V311G), SEQ ID NO: 19 (F104Q/S260A/V311A), SEQ ID NO: 20 (F104M/S260A/V311A), SEQ ID NO: 21 (F104Q/S260A/V311D), SEQ ID NO: 22 (F104A/S260A/V311A) and SEQ ID NO: 23 (F104Q/S260A/V311G). Preferably, the cytochrome P450 monooxygenase analog comprises or consists of an amino acid sequence according to SEQ ID NO: 23 (F104Q/S260A/V3111G).

In one embodiment, the cytochrome P450 monooxygenase analog consists of an amino acid sequence selected from the group consisting of SEQ ID NO: 2 (F84Q/V291A), SEQ ID NO: 3 (S240A/V291A), SEQ ID NO: 4 (F84A/V291A), SEQ ID NO: 5 (F84C/S240A/V291D), SEQ ID NO: 6 (F84C/S240A/V291A), SEQ ID NO: 7 (F84C/S240A/V291G), SEQ ID NO: 8 (F84Q/S240A/V291A), SEQ ID NO: 9 (F84M/S240A/V291A), SEQ ID NO: 10 (F84Q/S240A/V291D), SEQ ID NO: 11 (F84A/S240A/V291A), SEQ ID NO: 12 (F84Q/S240A/V291G), SEQ ID NO: 13 (F104Q/V311A), SEQ ID NO: 14 (S260A/V311A), SEQ ID NO: 15 (F104A/V311A), SEQ ID NO: 16 (F104C/S260A/V311D), SEQ ID NO: 17 (F104C/S260A/V311A), SEQ ID NO: 18 (F104C/S260A/V311G), SEQ ID NO: 19 (F104Q/S260A/V311A), SEQ ID NO: 20 (F104M/S260A/V311A), SEQ ID NO: 21 (F104Q/S260A/V311D), SEQ ID NO: 22 (F104A/S260A/V311A) and SEQ ID NO: 23 (F104Q/S260A/V311G).

### 3rd^{th} aspect - polynucleotide

In a third aspect, the invention relates to a polynucleotide encoding the cytochrome P450 monooxygenase analog of the second aspect. In a preferred embodiment, the polynucleotide encodes the cytochrome P450 monooxygenase analog of SEQ ID NO: 12.

### 4^{th} aspect - vector

In a fourth aspect, the invention relates to a vector comprising the polynucleotide encoding the cytochrome P450 monooxygenase analog of the third aspect, wherein the vector is preferably an expression vector.

### 5^{th} aspect - host cell

In a fifth aspect, the invention relates to a host cell comprising the cytochrome P450 monooxygenase analog of the third aspect and/or the polynucleotide of the third aspect, and/or the vector of the fourth aspect, wherein the host cell is preferably a bacteria cell, more preferred a bacteria cell belonging to the genus *Escherichia,* more preferred an *Escherichia coli* cell, more preferred an *Escherichia coli* cell selected from BL21 (DE3), BL21 (DE3) Gold, C41 (DE3) and C43 (DE3), more preferred *E. coli* C43 (DE3).

Below, the invention is described in more detail with respect to all aspects as mentioned above, especially in view of a process for preparing UDCA from LCA and in view of how the cytochrome P450 monooxygenase analogs of the third aspect were identified:
Techniques from protein engineering in combination with a whole-cell system to regenerate the cofactor NADPH required for P450 monooxygenases were used. A cytochrome P450 monooxygenase analog (OleP analog) was designed and an *Escherichia coli* (*E. coll*) based whole-cell system for the regio- and stereoselective hydroxylation of bile acids without a hydroxyl group in position 7 forming 7-beta-hydroxylated bile acid derivatives was created, which is shown for the transformation of LCA to UDCA in exemplarily manner in scheme 2 below:

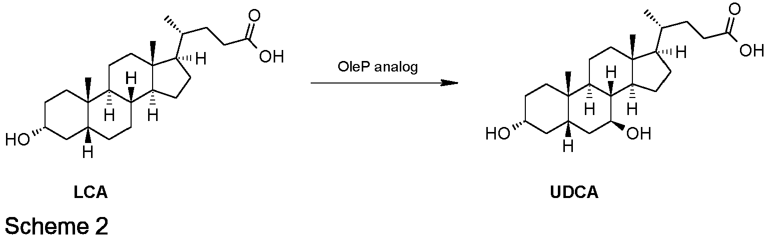

First, it was determined which amino acid residues could influence the positioning of the bile acid in the cytochrome P450 monooxygenase analog's active site. Using crystal structures of OleP with bound inhibitors and a natural substrate analog, it was possible to dock LCA to the OleP active site in multiple conformations. In this evaluation process a docked structure was found matching three desired criteria:
i) having LCA positioned at most 5 Å from the heme iron
ii) orientation of the 6-beta-hydrogen towards the heme, to mimic the binding of LCA for the intended MDCA formation, resulting in the elimination of perpendicular orientations, and
iii) since the steroid nucleus can flip in the horizontal orientation, the most frequently occurring conformations in the docking experiment was used. Based on this docking pose residues in a zone of 5-14 Å around the heme were chosen. Important P450 specificity-determining residues known from literature were also selected. Finally, residues interacting with the substrate analog were also selected.

In total 24 active site residues were identified for further investigations. "Residue" means an amino acid.

Alanine scanning was used to generate space in the active site, to allow LCA to shift from the 6-beta- towards 7-beta-hydroxylation and to investigate the contributions of residues to hydroxylation activity. Of the 24 identified active site residues, four were already alanine or glycine, the remaining 20 were exchanged to alanine by site-directed mutagenesis.

The generated cytochrome P450 monooxygenase analogs (in the following abbreviated as analogs or synonymously OleP analogs) were co-expressed with the redox partner proteins PdR/PdX in the *E.* coli strain C43 (DE3) and tested in whole-cell biotransformations. All generated cytochrome P450 monooxygenase analogs comprised a sequence based on the amino acid sequence of OleP but had in addition an initial Histag sequence and a linker sequence comprising 20 amino acids (MGSSHHHHHH SSGLVPRGSH). In the following, specific positions are mentioned with respect to OleP sequence - all analogs had 20 amino acids more, i.e. the position in the analog would be the specific position in the OleP sequence plus 20. Analysis by HPLC-RI revealed five UDCA-producing analogs (F84A, V93A, L94A, S240A and V291A). The analogs are indicated "letter-number-letter", wherein the one letter code of the amino acid of the parent cytochrome P450 monooxygenase CYP107D1 (OleP) from *Streptomyces antibioticus* is indicated first, followed by the number of the position within the amino acid sequence of OleP according to SEQ ID NO: 1, followed by the mutation, i.e. the indication of the amino acid being at that position in the analog.

L179A and S295A analogs produced no product and the remaining analogs produced MDCA, like the wild-type OleP. The analogs F84A, S240A and V291A were most promising as they produced mostly MDCA and UDCA compared to V93A and L94A, which produced several unidentified products.

The residue F84 of the BC loop is associated with the substrate recognition, S240 is part of the I helix and coordinates parts of the hydrogen-bond network, which connects OleP with the substrate similar to V93 and L94 of the BC loop, which coordinate water molecules in the active site. V291 is within a beta-hairpin (beta₃), part of a hydrophobic bulge, which coordinates the substrate by van der Waals interactions. This suggested that manipulation of the water-network and taking advantage of the hydrophobic interactions, which have significant effects in the positioning of the substrate, could enhance the 7-beta hydroxylation of LCA to form UDCA.

A 3DM (see R. K. Kuipers, H.-J. Joosten, W. J. H. van Berkel, N. G. H. Leferink, E. Rooijen, E. Ittmann, F. van Zimmeren, H. Jochens, U. Bornscheuer, G. Vriend et al., Proteins2010, 78, 2101) database of P450 monooxygenases was used to evaluate the most frequently occurring amino acids at each of the positions, which showed initial UDCA formation (F84A, V93A, L94A, S240A and V291A). To reduce the number of positions in the library as well as to keep the library size small enough for the screening of mutants, each residue was mutated to the four most frequently occurring amino acids as derived from the 3DM analysis. With these analogs created, UDCA formation and the number of side products formed were analyzed by HPLC. This narrowed down the residues to F84, S240 and V291 as analogs having mutations at these positions produced most UDCA.

Second, a "small but smart" 3DM library, i.e. a smaller 3DM library comprising a lesser number of analogs, was designed, which yielded in the past desired protein analogs while reducing the screening expenses: Position F84 was fully saturated, because this residue was not part of the 3DM core alignment, S240 was mutated to 14 residues (A, D, E, F, G, H, I, L, M, N, Q, S, T, V) and V291 was mutated to 16 residues (A, D, E, F, G, I, L, M, N, P, Q, R, S, T, V, W). This resulted in a library with 4,480 unique analogs. To eliminate bias, a high quality synthetic DNA library was produced by Twist Bioscience (see A. Li, C. G. Acevedo-Rocha, Z. Sun, T. Cox, J. L. Xu, M. T. Reetz, ChemBioChem2018, 19, 221).

The quality and diversity of the library after cloning into the target pET-28a vector and co-transformation with the redox partner system PdR/PdX into the *E. coli* C43 (DE3) expression host was confirmed by sequencing 96 clones. Only the desired codons were observed at each position and none of the amino acid substitutions were overrepresented.

The cultivation and biocatalytic reactions using whole-cells took place in a 24-deepwell plate format, which allowed standardization and semi-automatization of the process. Detection of the product initially depended on HPLC measurements.

Screening of libraries towards desired regio- and stereoselective hydroxylation of steroids and bile acids has, with some exceptions, mostly been done by chromatography-based methods. To shorten the analysis time per sample and to increase the screening throughput, a colorimetric UDCA assay was implemented. This assay employed an NADP⁺-dependent 7-beta-hydroxysteroid dehydrogenase (7beta-HSDH) from *Collinsella aerofaciens,* which converts UDCA to 7-ketolithocholic acid. In the process, NADP⁺ is converted to NADPH, which converted the tetrazolium salt WST-1 to a water-soluble formazan dye. UDCA formation was detected by monitoring the NADP⁺ dependent conversion of UDCA to 7-oxo LCA using a coupled assay. The electron coupling reagent 1-methoxy-5-methylphenazinium methyl sulfate (mPMS) reduces 2-(4-iodophenyl)-3-(4-nitrophenyl)-5-(2,4-disulfophenyl)-2H-tetrazolium (WST-1) to form the formazan dye. The reaction is shown schematically in Scheme 3 below:

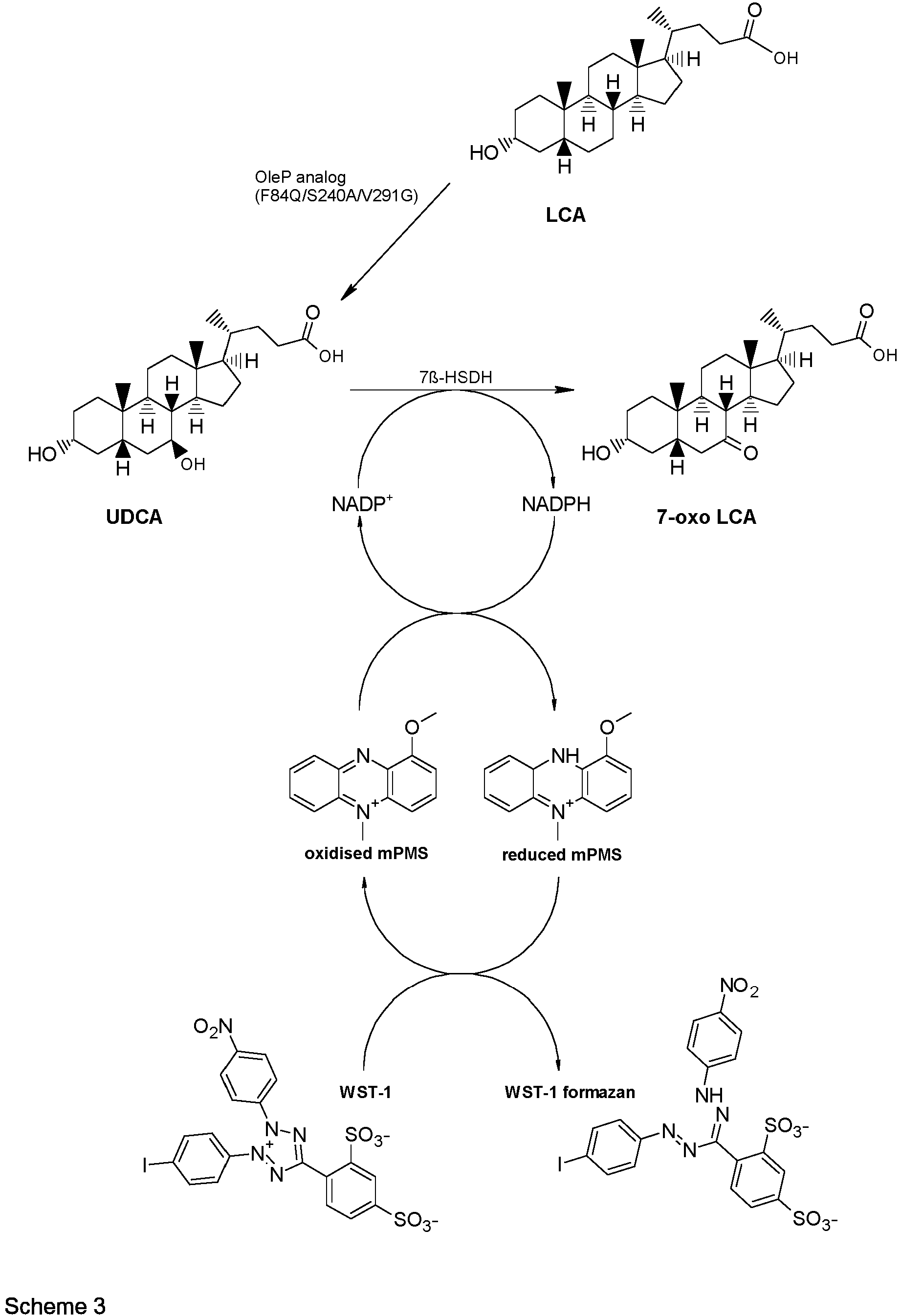

Using this colorimetric assay in 96 microtiter plates (MTPs), it was possible to increase the throughput drastically by screening 600 analogs per round.

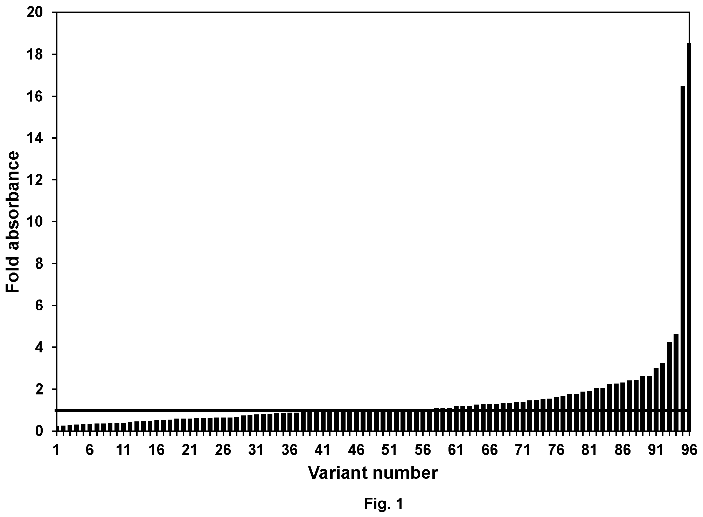

After screening a set of 3400 clones, analogs were found that gave an up to 18-fold increased absorbance, compared to the analog S240A, which showed the highest UDCA formation in the initial alanine scanning. S240A was used as benchmark/reference analog for the screening (Fig. 1).

To confirm these results, HPLC was used to rescreen the best 96 analogs identified using the colorimetric UDCA assay. UDCA production of these analogs was confirmed by comparison to a commercial UDCA standard. A clear correlation was observed between the colorimetric UDCA assay signals and the number of side products formed: an increased signal in the 7-beta-HSDH assay correlated with fewer side products as confirmed by HPLC. The analogs showing the highest colorimetric signal mostly produced MDCA and UDCA in different ratios with preferences towards UDCA ranging from 0.2 (F84Q/S240A), through 0.47 (F84C/S240A/V291A) up to 1.3 (F84Q/S240A/V291A). The specificity of F84Q/S240A/V291A for UDCA formation is remarkable, when considering that wildtype OleP produces only MDCA and the S240A analog had a UDCA:MDCA ratio of 0.09 (based on HPLC-data). None of the UDCA producing mutants showed the formation of the 7α-hydroxylation product (CDCA), demonstrating also the desired perfect stereoselectivity.

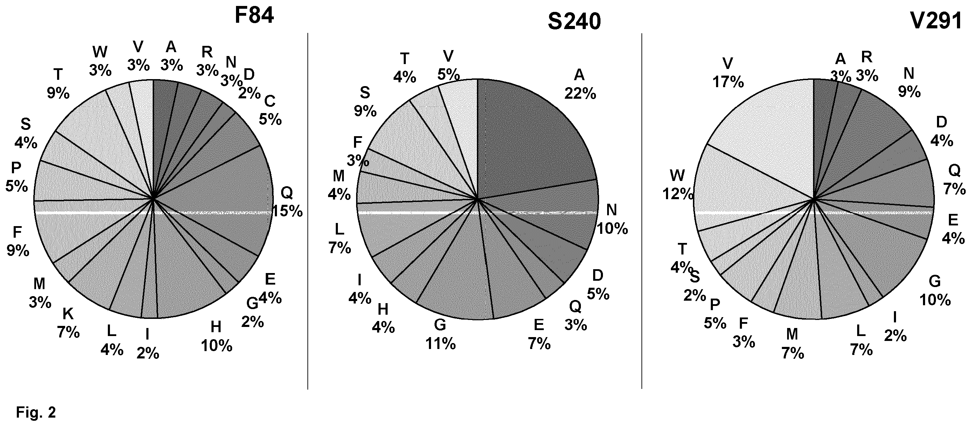

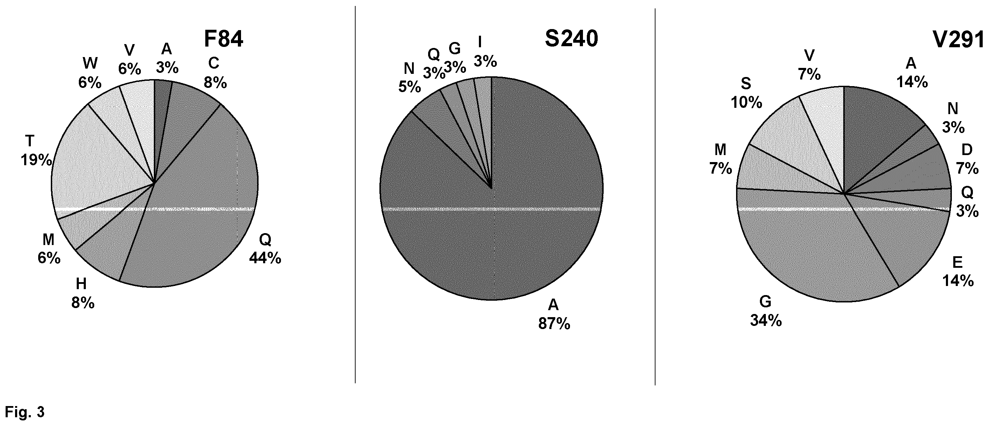

96 clones were sent for sequencing to verify the quality of the library with indicated amino acids at the positions F84, S240, V291, the sequencing results of 96 clones are shown in Fig. 2. The UDCA-producing analogs (Fig. 3) were sequenced and clear amino acid preferences were found at each of the randomized positions. In UDCA-producing analogs F84 was most frequently mutated to Q (44%) followed by T (19%), C (8%), and H (8%). For S240, a clear preference for A (87%) was observed and for V291 the most common analogs were G (34%), A (13%), E (10%), and S (10%).

It was hypothesized that the introduction of a polar and more flexible amino acid like Q, T and C at position F84 could enhance flexibility of the BC-loop, which is involved in substrate recognition and stabilization of the bile acid. Since S240 is a key part in the water network of the active site and is associated with the water-mediated coordination of substrates it was hypothesized that with the introduction of a nonpolar and small residue the water network in the active site is remodeled in favor towards the 7-beta-hydroxylation. As V291 is part of a hydrophobic cleft its mutation to glycine was speculated to be beneficial for the 7-beta-hydroxylation by the generation of space to position the more dented bile acid in the active site better compared to flat steroids such as testosterone.

To get further insights into the contribution of each position and to find the most selective analog, it was decided to create a set of 37 single, double and triple mutants containing the mutations F84 (Q, A, C, M), S240A and V291 (A, G, D). This selection was based on UDCA:MDCA ratios of the individual analogs (see Table 1) and was further supported by the sequencing results of the hit-mutants. All newly created analogs were analyzed by the colorimetric assay as well as by HPLC.

**Table 1: UDCA formation after 24 h whole cell reactions of the analogs generated for the structure function analysis.**

| Analog | UDCA formation [µM] | Analog | UDCA formation [µM] |
|---|---|---|---|
| S240A | 8.7 ± 3.1 | F84Q V291A | 8.5 ± 3.4 |
| F84A | 0.1 | F84Q V291 G | 4.2 |
| F84Q | 0.1 | F84Q V291D | 9.5 |
| F84C | 0.1 | F84C S240A | 0.1 |
| F84M | 3.7 | F84C V291A | 7.7 |
| V291A | 0.7 | F84C V291 G | 0.1 |
| V291G | 5.4 | F84C V291 D | 2.4 |
| V291D | 3.7 | F84M S240A | 0.1 |
| F84A S240A | 5.7 | F84M V291A | 0.1 |
| F84A V291A | 65.2 ± 3.2 | F84M V291G | 1.2 |
| F84A V291G | 0.1 | F84M V291D | 2.9 |
| F84A V291 D | 14 ± 2 | S240A V291 A | 18.2 ± 3.8 |
| F84Q S240A | 2.4 | S240A V291 D | 2.8 |
| S240A V291 G | 6.4 | F84A S240A V291 G | 4.2 |
| F84A S240A V291A | 58.5 ± 20.2 | F84A S240A V291 D | 3.2 |
| F84Q S240A V291A | 9.7 ± 3.1 | F84Q S240A V291 G | 67.1 ± 5.1 |
| F84Q S240A V291 D | 10.4 ± 3.1 | F84C S240A V291A | 7.2 ± 2.8 |
| F84C S240A V291 G | 9.2 ± 2.6 | F84C S240A V291 D | 5.6 ± 1.8 |
| F84M S240A V291A | 10.2 ± 2.6 | - | - |

Several analogs with single, double and triple mutations (Table 2) were able to produce up to 67 µM of UDCA although the selectivity towards UDCA varied significantly.

**Table 2: Structure-function analogs of OleP, which produce UDCA are displayed with the corresponding ratio of UDCA to MDCA. Reaction conditions: 2.5 mM LCA in 1 ml 200 mM potassium phosphate, 20 mM sodium chloride, 1% (w/v) glucose, and 0.4% (v/v) glycerol, pH 7.4 for 24 h at 220 rpm and 28°C.**

| Analog | UDCA formation µM (HPLC & 7-beta HSDH assay) | Ratio UDCA/MDCA (based on HPLC) |
|---|---|---|
| Wild-type* | - | Not indicated |
| S240A | 8.7 ± 3.1 | 0.09 |
| F84Q/V291A | 8.5 ± 3.4 | 0.2 |
| S240A/V291A | 18.2 ± 3.8 | 0.35 ± 0.06 |
| F84A/V291A | 65.2 ± 3.2 | 0.36 ± 0.06 |
| F84C/S240A/V291D | 5.6 ± 1.8 | 6.8 |
| F84C/S240A/V291A | 7.2 ± 2.8 | 0.47 ± 0.1 |
| F84C/S240A/V291G | 9.2 ± 2.6 | 0.76 ± 0.2 |
| F84Q/S240A/V291A | 9.7 ± 3.1 | 1.3 ± 0.5 |
| F84M/S240A/V291A | 10.2 ± 2.6 | 0.5 |
| F84Q/S240A/V291D | 10.4 ± 3.1 | 2.68 |
| F84A/S240A/V291A | 58.5 ± 20.2 | 0.49 ± 0.18 |
| F84Q/S240A/V291 G | 67.1 ± 5.1 | only traces of MDCA |

| | | |
|---|---|---|
| * cytochrome P450 monooxygenase CYP107D1 (OleP) from *Streptomyces antibioticus* | | |

Starting from the benchmark analog S240A, which produced mostly MDCA with a UDCA:MDCA ratio of 0.09, analogs, which produced UDCA and MDCA in equal amounts (F84C/S240AN291G or F84Q/S240A/V291A with ratios ~1.0) and more selective analogs like F84Q/S240AN291 D, with a ratio of 2.6, were identified. However, the most striking analog F84Q/S240A/V291G, which produced 67 µM of UDCA (conversion of 2.6%), was almost perfectly selective and produced only unquantifiable traces of MDCA as the only side-product.

Thus, it was possible to engineer OleP analogs, which exclusively formed MDCA, in a semi-rational manner towards UDCA production, resulting in the OleP analog F84Q/S240AN291G, which showed perfect stereoselectivity and excellent regioselectivity. This discovery opens new synthesis routes towards 7-beta-hydroxylated therapeutic agents like UDCA. These results also expand the knowledge about P450 mediated hydroxylations.

### Experimental Section

### Materials:

All chemicals were purchased from Sigma-Aldrich (St. Louis, USA) and used without further purification. Oligonucleotides were purchased from Thermo Fisher Scientific (Vantaa, Finland).

### Bacterial strains:

*Escherichia coliC43* (DE3), purchased from Lucigen (Middleton, USA), was used for expression and whole cell biotransformations. For plasmid amplification after site-directed mutagenesis, *E. coli* TOP10 or DH5α cells were used.

### Plasmids:

The plasmid containing the genes for the redox partners PdR and PdX were a gift from Prof. Anett Schallmey (Technical University Braunschweig, Braunschweig, Germany). A codon-optimized synthetic gene encoding CYP107D1 (UniProt entry Q59819) was purchased from GenScript and subcloned into the pET-28a vector using Ncol and Xhol restriction sites. The three-domain low-diversity combinatorial library of OleP (randomised at F84, S240, and V291) was purchased from Twist Bioscience and cloned into pET-28a using the restriction sites Ncol and Xhol.

### Site-directed Mutagenesis:

Site-directed mutagenesis was performed using the QuikChange method. PCRs contained 2.5 µl 10x Pfu⁺ buffer, 1.25 µl (5%) DMSO, 0.25 µM forward and reverse primer (each 1.25 µl), dNTPs (0.25 mM each), 50 ng of template (pET-28a-OleP), 0.4 µl Pfu⁺ polymerase and 17.35 µl ddH₂O. After initial denaturation for 30 s at 95°C, nineteen cycles of denaturation at 95°C for 30 s, annealing at 63°C (primers designed in order to fit all desired mutations to this temperature) for 30 s and extension at 72°C for 7 min were done followed by a final elongation step at 72°C for 10 min. 2 µl of the PCR mixture were used for heat shock transformation into *E. coli* Top10 or DH5α cells, cells were grown on LB agar plates containing 25 µg/ml kanamycin overnight at 37°C. Three colonies of each mutant were picked, cultured in LB (25 µg/ml kanamycin) at 37°C, 220 rpm for 10 h, followed by plasmid isolation and sequencing. Confirmed mutants were co-transformed with *pACYC-camA*/*B* into *E. coli C43* (DE3) for further studies and biotransformations.

### Library creation:

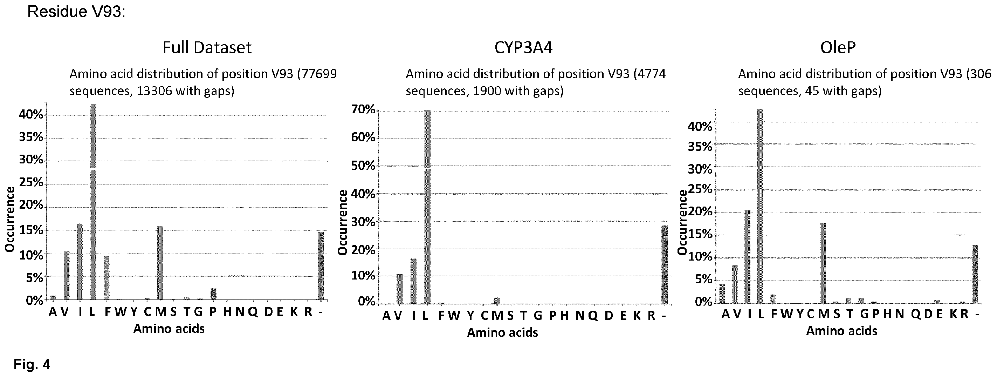

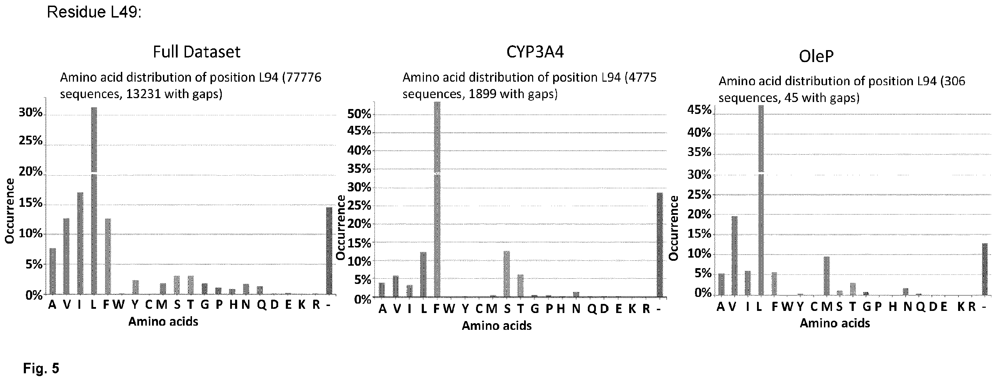

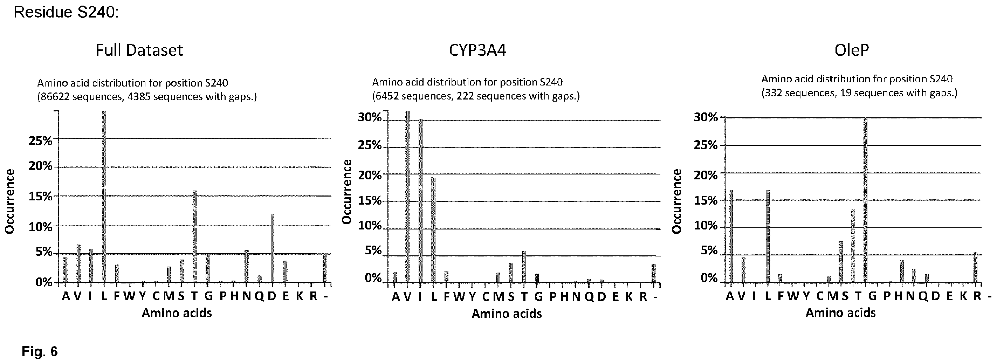

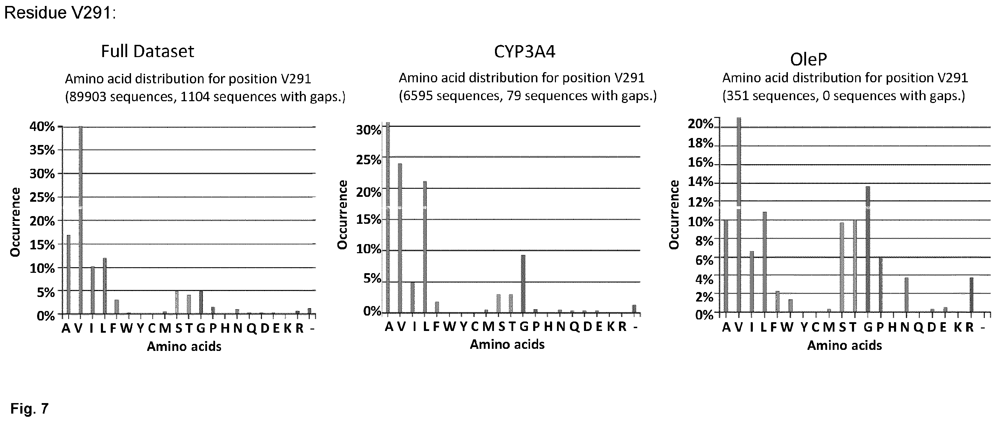

The synthetic OleP library was ordered codon-optimized as gene strings at Twist Bioscience. Residue F84 was fully saturated, while S240 (A,D,E,F,G,H,I,L,M,N,Q,S,T,V) and V291 (A,D,E,F,G,I,L,M,N,P,Q,R,S,T,V,W) were varied to residues naturally occurring at these positions (see 3DM analysis in Fig. 4-7). The flanking sequences of the gene prior to the start codon was CCCGTCACCTTTGGCTTATCAGT-AGAAGGAGATATACC. After the stop codon the sequence TGACTCGAGCACCAC AGTGACATCTGGACGCTAAGACCG was added. The flanking regions included the restriction sites for Ncol and Xhol. The library was cloned into pET28-a using the restriction sites Ncol and Xhol.

The vector was digested in a reaction containing 1x CutSmart^{®} buffer, 579 ng pET-28a vector template vector (concentration of 57.9 ng/µl), 1 µl Xhol, 1 µl Ncol-HF (high fidelity), 23 µl ddH₂O in a final volume of 25 µl. For the digestion of the insert 5 µl CutSmart buffer were used, 1 µl Xhol, 1 µl Ncol-HF, 7.5 µl OleP library (150 ng) and 35.5 µl ddH₂O. The digestion was done for 16 h at 37°C, followed by heat inactivation at 80°C for 20 min. The digested vector was separated on a 1% (w/v) agarose gel and isolated using a gel purification kit (Macherey-Nagel). The library was isolated after the double digest by PCR clean up.

The ligation of vector and insert was done in a 30 µl reaction containing 10 µl of digested pET-28a vector (100 ng) and 8.8 µl insert (75.38 ng), 3 µl of 10x ligase buffer, 1.5 µl T4-ligase and 6.7 µl ddH₂O. The ratio of vector and insert was calculated using NEB calculator (https://nebiocalculator.neb.com/#!/ligation) and was 1:3. The ligation mix was heat-inactivated at 70°C for 15 min and dialysed against ddH₂O. The pET-28a OleP library was co-transformed with the reductase system CamA/B on the pACYC vector by electroporation into *E. coliC43* (DE3) using molar ratios of 1:8^{[34]}. A picking robot (QPix 420, Molecular Devices) was used to transfer 10,560 clones into 96-well microtiter plates containing 150 µl LB medium per well (supplemented with 25 µg/ml kanamycin and 25 µg/ml chloramphenicol). Cultures were grown at 37°C and 250 rpm for 16 h and glycerol stocks were prepared by adding 150 µl of 60% (v/v) glycerol to each well (final glycerol concentration was 30% v/v).

Table 3 shows the library design used for synthetic library.

**Table 3: library design used for synthetic library.**

| Variant | 1 | 2 | 3 |
|---|---|---|---|
| AA (position) - ORF | 104 | 260 | 311 |
| AA | (F) | (S) | (V) |
| bp (start) - ORF | 310 | 778 | 931 |
| Codon | TTC | AGT | GTT |
| A | A | A | A |
| C | C | | |
| D | D | D | D |
| E | E | E | E |
| F | F | F | F |
| G | G | G | G |
| H | H | H | |
| I | I | I | I |
| K | K | | |
| L | L | L | L |
| M | M | M | M |
| N | N | N | N |
| P | P | | P |
| Q | Q | Q | Q |
| R | R | | R |
| S | S | S | S |
| T | T | T | T |
| V | V | V | V |
| W | W | | W |
| Y | Y | | |
| Variant AA's | 20 | 14 | 16 |

### Expression and purification of recombinant Collinsella aerofaciens 7-beta-HSDH:

Initially, the 7beta-hydroxysteroid dehydrogenases (7beta-HSDH) from *Clostridium absonum* and *Collinsella aerofaciens* were considered. The *C. absonum* enzyme has a high specific activity (89 U/mg) and about 24 mg can be expressed per liter of culture. The *C. aerofaciens* enzyme has a lower specific activity (17 U/mg) but about 300 mg can be expressed per liter of culture. Most importantly, the enzyme can be stored at -20°C for several months, not losing activity after more than 100 freeze-thaw cycles.

A synthetic gene encoding the *C. aerofaciens* 7-beta-HSDH (GenBank accession number, ZP_01773061.1), subcloned into pET-28a(+), was ordered from BioCat GmbH (Heidelberg, Germany). *E. coli* BL21(DE3) was transformed using approximately 50 ng of the expression vector and plated on LB agar containing 50 µg/ml kanamycin and 1% glucose. After overnight incubation at 37°C, single colonies were used to inoculate three 2 l flasks, each containing 200 ml of autoinduction medium (TB medium supplemented with 0.05% glucose, 0.2% lactose, and 100 µg/ml kanamycin). Cultures were incubated at 30°C and 200 rpm for about 24 hours. The three 200 ml cultures were pooled and the cells harvested by centrifugation at 4500 g and 4°C for 30 min. Cells were resuspended in 20 ml LEW buffer (300 mM sodium chloride, 50 mM sodium phosphate, pH 8.0) and lysed by a single pass through a French press. Crude lysate was cleared by centrifugation at 10,000 *g* and 4°C for one hour. The clarified lysate was applied to a 2 ml column of Protino Ni-TED resin preequilibrated with LEW buffer. After washing the column with 20 ml of LEW buffer, 10 ml of elution buffer (LEW containing 250 mM imidazole) was applied to each column and the eluate was collected in ~1 ml fractions. Fractions containing the most protein were identified by measuring absorbance at 280 nm and pooled. An equal volume of 80% (v/v) glycerol was added before storage at -20°C.

### UDCA assay using the recombinant Collinsella aerofacions 7-beta-HSDH:

The UDCA produced by OleP analogs was quantified using a spectrophotometric assay. The NADP⁺-dependent 7-beta-hydroxysteroid dehydrogenase (7-beta-HSDH) from *Collinsella aerofaciens was* used to convert UDCA and NADP⁺ to 7-ketolithocholic acid and NADPH, respectively. NADPH formation was coupled to the reduction of the tetrazolium salt WST-1 using 1-methoxyphenazine methosulfate (mPMS). Reduction of WST-1 results in formation of a water-soluble yellow formazan dye (ε^{455nm} = 34,660 M⁻¹cm⁻¹).

Samples (200 µl) of biotransformations using the OleP analogs were transferred to 96-well microtiter plates. Because this crude supernatant gives significant background absorbance at 450 nm, 7-beta-HSDH-independent absorbance was measured before 7-beta-HSDH was added and a second absorbance measurement was performed. To each 200 µl sample, 40 µl of a master mix giving final concentrations of 40 mM Tris-HCl (pH 7.5), 100 µM NADP⁺, 10 µM mPMS, and 100 µM WST-1 was added. Plates were gently shaken for five to ten minutes before absorbance at 450 nm was measured using a Varioskan^{™} LUX plate reader (Thermo Fisher Scientific, Vantaa, Finland). Next, 10 µl (40 µg) of 7-beta-HSDH (2.5 mg/ml in 40% v/v glycerol) was added. Plates were gently shaken for about 10 min before absorbance at 450 nm was measured again. A 7-beta-HSDH-dependent increase in absorbance between the two measurements reflected the presence of UDCA in the sample. To allow UDCA quantification a standard curve was set up. A series of UDCA concentrations (0, 5, 10, 20, 30, 40, 50, 60, 70, 80, 90, and 100 µM) was prepared in control biotransformation supernatant harboring only the reductase system CamA/B on a pACYC vector, closely mimicking the screening conditions. The 40 µl assay master mix was added and plate was shaken for about 5 min before the first absorbance measurement was taken. The 7-beta-HSDH was then added and the plate gently shaken for about 10 min before taking the second absorbance measurement. The difference in absorbance (450 nm) was calculated and plotted against UDCA concentration. A linear relationship (R² = 0.99) between absorbance at 450 nm and UDCA concentration was observed, with a detection limit of 6 µM. This method allowed accurate quantification of UDCA formation despite significant variation in initial absorbance values, which presumably results from leakage of cellular NADH into the resting cell medium (200 mM potassium phosphate, 20 mM sodium chloride, 1% (w/v) glucose, and 0.4% (v/v) glycerol, pH 7.4).

### Whole-cell biocatalysis:

The whole-cell biocatalysis was performed similar to Grobe et al. 2020 (S. Grobe, A. Wszo 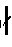 ek, H. Brundiek, M. Fekete, U. T. Bornscheuer, *Biotechnol. Lett.* 2020, 819). Starter cultures were prepared by inoculating 96-well microtiter plates containing 240 µl LB medium per well (supplemented with 25 µg/ml kanamycin and 25 µg/ml chloramphenicol) using the library glycerol stocks. Plates were sealed using air permeable membranes and grown for 12-16 h at 37°C and 250 rpm (Infors Multitron Standard, Bottmingen, Switzerland).

Prewarmed TB medium (2 ml per well of a 24-deepwell pate, supplemented with 25 µg/ml kanamycin and 25 µg/ml chloramphenicol) was inoculated with 1% (v/v) of the starter culture and grown at 37°C for 4-5 h until an OD₆₀₀ of 1 was reached. The plates were sealed with air permeable membranes. The cultures were cooled to 28°C and supplemented with 0.64 mM 5-aminolevulinic acid and 0.3 mM FeSO₄, followed by induction of protein expression using 0.4 mM IPTG (isopropyl-beta-D-thiogalactoside) and incubation for 16 h at 28°C and 250 rpm (Infors Multitron Standard, Bottmingen, Switzerland). Cells were harvested by centrifugation (3000 g, 30 min, 4°C), the supernatant was discarded, and the cells were washed with resting cell medium (200 mM potassium phosphate, 20 mM sodium chloride, 1% (w/v) glucose, and 0.4% (v/v) glycerol, pH 7.4) followed by resuspension in 1 ml of resting cell medium (final OD₆₀₀ ≈ 30). LCA was added to a final concentration of 5 mM (2 mg/ml) for the initial screening procedure and 2.5 mM (1 mg/ml) for tests of individual analogs from a 100 mg/ml stock solution in DMSO. Reactions were incubated at 28°C and 250 rpm for 24h. The reactions were stopped by centrifugation (3,000 *g,* 30 min, 4 °C) followed by transfer of the supernatant (1 ml) to a 96-deepwell plate. The supernatants were used for quantification of the UDCA produced either by HPLC or the 7beta-HSDH assay.

### HPLC analysis:

The whole-cell biocatalysis was following the general protocol of Grobe et al. 2020 (S. Grobe, A. Wszo 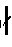 ek, H. Brundiek, M. Fekete, U. T. Bornscheuer, *Biotechnol. Lett.* 2020, 819). Samples (500 µl) were extracted twice using 1 ml ethyl acetate. The combined organic phases were dried over anhydrous sodium sulfate. After complete evaporation of the ethyl acetate, the residue was dissolved in 250 µl ethanol. Samples (10 µl) were then analyzed using a Hitachi LaChrom Elite HPLC system (Hitachi High-Technologies Europe GmbH, Krefeld, Germany) equipped with a Luna Omega 5 µm PS C18 100Å LC column (Phenomenex LTD, Aschaffenburg, Germany). The mobile phase was acetonitrile:water 1:1 (v/v) containing 0.1% trifluoroacetic acid. An isocratic method (1 ml/min) was employed at 40°C oven temperature. The bile acids were detected using a LaChrom Elite L-2490 Refractive Index Detector, which was heated to 40°C (Hitachi High-Technologies Europe GmbH, Krefeld, Germany).

### Determination of the P450 concentration:

The concentration of P450 monooxygenase was determined as described by Omura and Sato (see T. Omura, R. Sato, J. Biol. Chem. 1964, 239, 2370). To a 2 ml lysate sample 2 µM Safranin T (in 50 mM phosphate buffer pH 7.5) were added, followed by the addition of a spatula tip of sodium dithionite (see F. P. Guengerich, M. V. Martin, C. D. Sohl, Q. Cheng, Nature Prot. 2009, 4). The tube was inverted and split into 2 samples each with 1 ml. To one sample carbon monoxide (CO) was bubbled with 1 bubble per second for one minute. A CO-differential spectrum (Jasco V-550, Pfungstadt, Germany) was recorded between 400 and 500 nm and the concentration of the P450 monooxygenase was calculated.

### Molecular docking:

For the molecular docking experiments the structures of OleP in complex with clotrimazol (PDB-code: 4XE3) and OleP in complex with 6-deoxyerythronolide B (PDB-code: 5MNS) were used together with YASARA (Vienna, Austria) followed by display and structural analysis in Chimera (see E. F. Pettersen, T. D. Goddard, C. C. Huang, G. S. Couch, D. M. Greenblatt, E. C. Meng, T. E. Ferrin, J. Comp. Chem. 2004, 25, 1605). Prior to the docking experiments water and ligands were removed from the PDB files and the simulation cell moved to the heme cofactor. The compounds to dock were LCA (PDB-code: 4OA) and testosterone. Afterwards, the dock_run and dockrunensemble macros from YASARA were used with its standard settings for all docking experiments. The resulting clusters were viewed with chimera and compared to proper positioning of the B-ring of the steroid in 5 Å distance towards the heme center and a horizontal orientation in binding towards the heme cofactor. The results for LCA were compared with the docking experiments for testosterone and residues for the alanine scanning picked based on a zone of 5-11 Å together with substrate coordinating residues extracted from the OleP structures (PDB-codes: 4XE3, 5MNS) and substrate recognition sites in P450 monooxygenases (see O. Gotoh, J. Biol. Chem. 1992, 1992, 81).

### 3DM analysis:

The distribution of the amino acids at the positions F84, V93, L94, S240, V291 was determined using the 3DM platform from the company Bio-Prodict (Nijmegen, The Netherlands) based on a P450 monooxygenase superfamily database. For comparison with the overall dataset two subsets originating from the P450 monooxygenase superfamily were build and used. One of these corresponds to OleP (PDB-code: 4XE3A) and the other towards the human P450 monooxygenase CYP3A4. The resulting distributions at each position are displayed in Figures 4 to 7. Based on this analysis, the amino acids substitutions for the library were chosen for site-directed mutagenesis.

### 3DM analysis of the residues F84, V93, L94, S240 and V291:

F84 is not part of the core region, which is used for the 3DM alignment, therefore, here are no data are available. CYP3A4 as prominent member of P450s with a broad substrate scope was used as second reference besides the overall dataset.

### Alanine scanning:

The residues picked for the alanine scanning were: F84, E89, V93, L94, L179, S240, I243, H246, E247, T248, S249, L290, V291, S292, S295, F296, F321, M395, I397. The residues G82, A95, A244, G294 were also within the 14 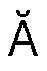 zone around the heme but were not mutated to alanine.

### Primers:

The primer list for mutations implemented by site directed mutagenesis is indicated below in Table 4.

**Table 4: Primer list for mutations implemented by site-directed mutagenesis.**

| | |
|---|---|
| CAACCCCGCGTATGGCGCCGACCCCGCCGG | F84A_Fwd |
| CCGGCGGGGTCGGCGCCATACGCGGGGTTG | F84A_Rev |
| CGACCCCGCCGGCCCCGGATGGTGTTC | E89A_Fwd |
| GAACACCATCCGGGGCCGGCGGGGTCG | E89A_Rev |
| GAACCGGATGGTGCGCTGGCCCAGGATC | V93A_Fwd |
| GATCCTGGGCCAGCGCACCATCCGGTTC | V93A_Rev |
| CCGGATGGTGTTGCGGCCCAGGATCC | L94A_Fwd |
| GGATCCTGGGCCGCAACACCATCCGG | L94A_Rev |
| CAGCGATGCGATGGCGAGCTCTACCCG | L179A_Fwd |
| CGGGTAGAGCTCGCCATCGCATCGCTG | L179A_Rev |
| GTGAATATGGGTGTTGCGCTGCTGATCGCCGG | S240A_Fwd |
| CCGGCGATCAGCAGCGCAACACCCATATTCAC | S240A_Rev |
| GGTGTTAGTCTGCTGGCGGCCGGTCATGAAAC | I243A_Fwd |
| GTTTCATGACCGGCCGCCAGCAGACTAACACC | I243A_Rev |
| GCCGGTCATGAAGCGTCCGTGAACC | T248A_Fwd |
| GGTTCACGGACGCTTCATGACCGGC | T248A_Rev |
| CGCTACACGCCGGCGGTTTCAGCGG | L290A_Fwd |
| CCGCTGAAACCGCCGGCGTGTAGCG | L290A_Rev |
| CACGCCGCTGGCGTCAGCGGGCTCG | V291A_Fwd |
| CGAGCCCGCTGACGCCAGCGGCGTG | V291A_Rev |
| GTTTCAGCGGGCGCGTTTGTTCGTG | S295A_Fwd |
| CACGAACAAACGCGCCCGCTGAAAC | S295A_Rev |
| CAGCGGGCTCGGCGGTTCGTGTCGCC | F296A_Fwd |
| GGCGACACGAACCGCCGAGCCCGCTG | F296A_Rev |
| GTGCGTGGTTCACGCGGCATCGGCTAACC | F321A_Fwd |
| GGTTAGCCGATGCCGCGTGAACCACGCAC | F321A_Rev |
| GAAACAGGGTATGGCGATTCGCGGTCTGG | L396A_Fwd |
| CCAGACCGCGAATCGCCATACCCTGTTTC | L396A_Rev |
| GAAACAGGGTATGCTGGCGCGCGGTCTGGAACGTC | I397A_Fwd |
| GACGTTCCAGACCGCGCGCCAGCATACCCTGTTTC | I397A_Rev |
| GACCGGCGATCAGCAGCACAACACCCATATTCAC | S240V_Rev |
| GAATATGGGTGTTATTCTGCTGATCGCCGG | S240I_Fwd |
| CCGGCGATCAGCAGAATAACACCCATATTC | S240I_Rev |
| GTGAATATGGGTGTTCTGCTGCTGATCGCCGGTC | S240L_Fwd |
| GACCGGCGATCAGCAGCAGAACACCCATATTCAC | S240L_Rev |
| GTGAATATGGGTGTTGGCCTGCTGATCGCCGG | S240G_Fwd |
| CCGGCGATCAGCAGGCCAACACCCATATTCAC | S240G_Rev |
| GAATATGGGTGTTACTCTGCTGATCGCCG | S240T_Fwd |
| CGGCGATCAGCAGAGTAACACCCATATTC | S240T_Rev |
| GAACCGGATGGTCTTCTGGCCCAGG | V93L_Fwd |
| CCTGGGCCAGAAGACCATCCGGTTC | V93L_Rev |
| GAACCGGATGGTATTCTGGCCCAGG | V93I_Fwd |
| CCTGGGCCAGAATACCATCCGGTTC | V93I_Rev |
| CGGAACCGGATGGTATGCTGGCCCAGGATCC | V93M_Fwd |
| GGATCCTGGGCCAGCATACCATCCGGTTCCG | V93M_Rev |
| GAACCGGATGGTGGCCTGGCCCAGGATC | V93G_Fwd |
| GATCCTGGGCCAGGCCACCATCCGGTTC | V93G_Rev |
| GAACCGGATGGTGTTTTTGCCCAGGATCCGCC | L94F_Fwd |
| GGCGGATCCTGGGCAAAAACACCATCCGGTTC | L94F_Rev |
| GAACCGGATGGTGTTATTGCCCAGGATCCGCC | L94I_Fwd |
| GGCGGATCCTGGGCAATAACACCATCCGGTTC | L94I_Rev |
| CCGGATGGTGTTGTGGCCCAGGATC | L94V_Fwd |
| GATCCTGGGCCACAACACCATCCGG | L94V_Rev |
| GAACCGGATGGTGTTAGCGCCCAGGATCCGCC | L94S_Fwd |
| GGCGGATCCTGGGCGCTAACACCATCCGGTTC | L94S_Rev |
| CCCCGCGTATGCAGCCGACCCCGCC | F84Q_Fwd |
| GGCGGGGTCGGCTGCATACGCGGGG | F84Q_Rev |
| CCCCGCGTATGTGCCCGACCCCGCC | F84C_Fwd |
| GGCGGGGTCGGGCACATACGCGGGG | F84C_Rev |
| CAACCCCGCGTATGGCGCCGACCCCGCCGG | F84A_Fwd |
| CCGGCGGGGTCGGCGCCATACGCGGGGTTG | F84A_Rev |
| CCCCGCGTATGATGCCGACCCCGCCG | F84M_Fwd |
| CGGCGGGGTCGGCATCATACGCGGGG | F84M_Rev |
| GTGAATATGGGTGTTGCGCTGCTGATCGCCGG | S240A_Fwd |
| CCGGCGATCAGCAGCGCAACACCCATATTCAC | S240A_Rev |
| CACGCCGCTGGCGTCAGCGGGCTCG | V291A_Fwd |
| CGAGCCCGCTGACGCCAGCGGCGTG | V291A_Rev |
| CACGCCGCTGGGCTCAGCGGGCTCG | V291G_Fwd |
| CGAGCCCGCTGAGCCCAGCGGCGTG | V291G_Rev |
| CTACACGCCGCTGGATTCAGCGGGCTCG | V291D_Fwd |
| CGAGCCCGCTGAATCCAGCGGCGTGTAG | V291D_Rev |

### DNA sequence of the OleP library with attached linker regions (underlined), mutation sites (bold and underlined)

AGAAGGAGATATACCatgggcagcagccatcatcatcatcatcacagcagcggcctggtgccgcgcggcagccatat
gaccgatacgcataccggcccgaccccggcagacgcagtcccggcatacccgtttagcctgccgcacgccctggacctggac
ccgcattatgcggaactgcgtcgcgatgaaccggtcagccgtgtgcgtctgccgtacggcgagggtacggcatggctggtcacc
cgcatgtctgacgctcgtattgtgctgggcgatagtcgcttctccaccgccgcggcaaccgatccggcaaccccgcgtatgTTCc
cgaccccgccggaaccggatggtgttctggcccaggatccgccggaccatacccgtctgcgtcgcctggttggtaaagcgtttac
cgcccgtcgcgtcgaagaaatgcgtccgcgcgttcgtagcctggtcgactctctgctggatgacatggttgcgcacggcagtccg
gccgatctggttgaatttctggcagtcccgttcccggtcgctgtgatttgcgaactgctgggtgtgccgctggaagatcgcgacctgtt
tcgtacgttcagcgatgcgatgctgagctctacccgcctgacggctgcggaaatccagcgtgtgcagcaagactttatggtttatat
ggatggcctggtcgcacaacgtcgcgatgctccgaccgaagatctgctgggtgctctggcgctggccacggataacgatgacca
tctgaccaaaggcgaaattgtgaatatgggtgttAGTctgctgatcgccggtcatgaaacgtccgtgaaccagatcaccaatctg
gttcacctgctgctgaccgaacgcaaacgttatgaatctctggttgcagatccggctctggtcccggcagcagtggaagaaatgct
gcgctacacgccgctgGTTtcagcgggctcgtttgttcgtgtcgccaccgaagacgtggaactgtcaaccgtgaccgttcgtgcg
ggtgaaccgtgcgtggttcacttcgcatcggctaaccgtgatgaagaagtgtttgatcatgcagacgaactggatttccaccgcga
acgtaatccgcatattgcgtttggccacggtgcccatcactgtatcggcgcccagctgggtcgtctggaactgcaagaagcgctga
gcgcactggttcgtcgcttcccgaccctggatctggcagaaccggtggctggtctgaaatggaaacagggtatgctgattcgcggt
ctggaacgtcaaatcgtgagctggtaaTGACTCGAGCACCAC

Furthermore, additional flanking regions were incorporated by TwistBioscience:
CCCGTCACCTTTGGCTT A TCAGTnnn... nnnAGTGACA TCTGGACGCT AAGACCG

Amino acid sequence of the OleP library (mutation sites indicated in parenthesis) including 20 initial amino acids (Histag + linker, underlined)

MGSSHHHHHHSSGLVPRGSHMTDTHTGPTPADAVPAYPFSLPHALDLDPHYAELRRDEPV
SRVRLPYGEGTAWLVTRMSDARIVLGDSRFSTAAATDPATPRM(F)PTPPEPDGVLAQDPPD
HTRLRRLVGKAFTARRVEEMRPRVRSLVDSLLDDMVAHGSPADLVEFLAVPFPVAVICELLG
VPLEDRDLFRTFSDAMLSSTRLTAAEIQRVQQDFMVYMDGLVAQRRDAPTEDLLGALALAT
DNDDHLTKGEIVNMGV(S)LLIAGHETSVNQITNLVHLLLTERKRYESLVADPALVPAAVEEML
RYTPL(V)SAGSFVRVATEDVELSTVTVRAGEPCVVHFASANRDEEVFDHADELDFHRERNP
HIAFGHGAHHCIGAQLGRLELQEALSALVRRFPTLDLAEPVAGLKWKQGMLIRGLERQIVSW

### Preparative scale reaction:

Whole-cell biocatalysis was performed with the OleP analog according to SEQ ID NO 23 (F104Q/S260A/V311 G, i.e. F84Q/S240A/V291 G in view of the amino acid sequence as in SEQ ID NO 12 without Histag, linker) as described in Grobe et al. 2020 (S. Grobe, A. Wszo 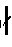 ek, H. Brundiek, M. Fekete, U. T. Bornscheuer, *Biotechnol. Lett.* **2020,** 819). 500x 1 ml reactions were set up and performed as described.

### Product isolation and NMR verification:

500 samples were extracted twice using 1 ml ethyl acetate. The combined organic phases were dried over anhydrous sodium sulfate. The organic solvent was evaporated, the residue dissolved in 10 ml ethanol and applied to preparative and analytical HPLC measurements. Preparative and analytical HPLC were performed using the Shimadzu devices CBM-20A, LC-20A P, SIL-20A, FRC-10A with an ELSD-LTII detector. A semi-preparative post-column adjustable flow splitter (Analytical Scientific Instruments) was utilized for evaporative light scattering detection in preparative mode. In analytical mode a LiChroCART^{®} (250×4 mm, Merck) and in preparative mode a Hibar^{®} RT (250×25 mm, Merck) column, both containing LiChrospher^{®} 100 RP-18e (5 µm), were used. The UDCA product was separated from impurities and remaining substrate by preparative HPLC. The fractions containing the UDCA product were pooled, the solvents evaporated and the residue dried *in vacuo.* The dried residue was dissolved in 500 µl deuterated methanol and applied for NMR-spectroscopy.

### NMR-spectroscopy:

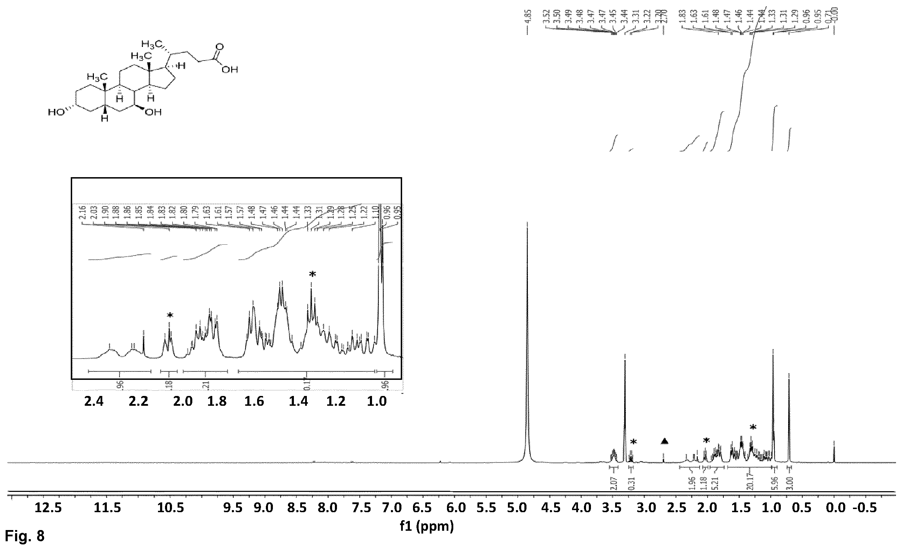

For the calibration of the ¹H and ¹³C NMR measurements, tetramethylsilane was used. The purified UDCA was dissolved in deuterated methanol and measured at 400 MHz for ¹H and 101 MHz for ¹³C measurements. To assign protons and carbon atoms, 2D NMR techniques (H,H-COSY, HSQC, HMBC) were used. The ¹H-NMR spectrum of the isolated product, which was identified as UDCA, is shown in Fig. 8.

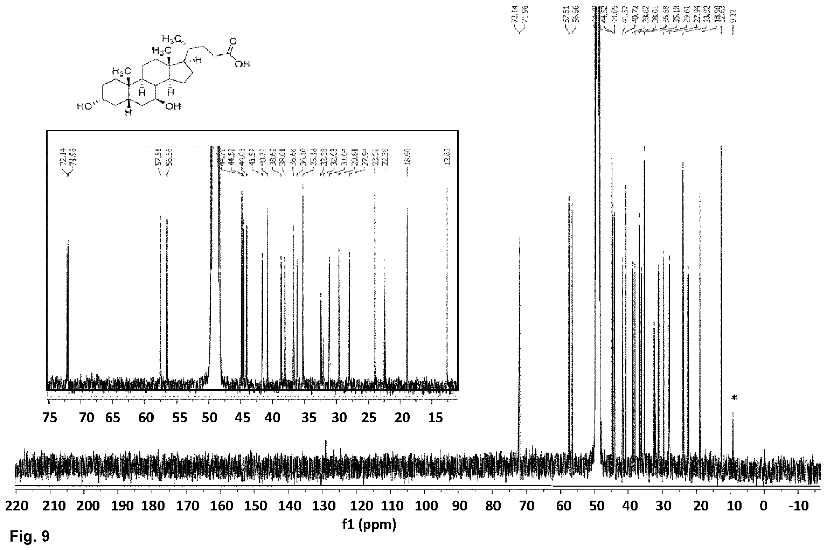

The ¹H-NMR spectrum of the isolated fraction can be unambiguously assigned to UDCA. Minor additional signals found in the ¹H-NMR spectrum, which do not originate from the target molecule are marked with a asterisk (*) or a triangle (▲). The asterisk indicates signals assigned to a methyl ethyl ketone contamination. The signal marked with a triangle can be assinged to minor amounts of DMSO. Notably, no signals for other hydroxylated bile acid species were observed. Using 2D NMR techniques (H,H-COSY, HSQC, HMBC) the signals for the target molecule UDCA as well as contaminations could be assigned to the corresponding signals in the ¹³C-NMR spectrum, here again the signal for the contamination of methyl ethyl ketone is indicated with a asterisk. The signal for the carboxylic acid function at 178 ppm in the ¹³C-NMR spectrum is not clearly observed, which is expected when using deuterated methanol as solvent. Peak broadening, even diminishing, of carboxylic acid functional groups can be attributed to the Nuclear Overhauser effect in NMR-spectroscopy. The ¹H-NMR spectrum together with the ¹³C-NMR spectrum (see Fig. 9) supported by the 2D spectra clearly identified the isolated product as UDCA.

**¹H-NMR, H,H-COSY (400 MHz, CD₃OD):** δ (ppm) = 3.54-3.42 (m, 2H), 2.41-2.12 (m, br, 2H), 2.08-1.99 (m, 2H), 1.96-1.74 (m, 5H), 1.67-0.98 (m, 19H), 0.96 (s, 3H), 0.95 (s, 3H), 0.71 (s, 3H); **¹³C-NMR, DEPT135, HSQC, HMBC (101 MHz, CD₃OD):** δ (ppm) =72.1 (7), 72.0 (3), 57.5 (17), 56.6 (14), 44.8 (13), 44.5 (8), 44.1 (5), 41.6 (12), 40.7 (9), 38.6 (4), 38.0 (6), 36.7 (20), 36.1 (1), 35.2 (10), 32.4 (22), 32.0 (23), 31.0 (2), 29.6 (16), 27.9 (15), 23.9 (19), 22.4 (11), 18.9 (21), 12.6 (18)

### Description of Figures

- **Fig. 1**: shows the screening results using the 7-beta-HSDH assay. Analogs showing up to 18-fold increased absorbance compared to the positive control OleP S240A (indicated by the horizontal line at fold absorbance = 1) were identified.
- **Fig. 2**: shows the sequencing results of 96 clones sent for sequencing to verify the quality of the library with indicated amino acids at the positions F84, S240, V291.
- **Fig. 3**: shows the sequencing results of the by HPLC-RI in the rescreening confirmed analogs, which produce UDCA and the distribution of each amino acid at the three position F84, S240, V291
- **Fig. 4**: shows the 3DM analysis of residue V93, which is assigned as core position 56 in the 3DM database for P450 monooxygenase analogs with detailed occurrence of each of the amino acids at this position. Here, three different sub datasets are shown: the full dataset to the left covering 77,699 sequences of which 13,306 have gaps followed by the subset used for comparisons based on CYP3A4 harboring 4,774 sequences of which 1,900 have gaps and to the right the subdataset of OleP analogs with 306 sequences of which 45 have gaps.
- **Fig. 5**: shows the 3DM analysis of residue L94, which is assigned as core position 57 in the 3DM database for P450 monooxygenase analogs with detailed occurrence of each of the amino acids at this position. Here, three different sub datasets are shown: the full dataset to the left covering 77,776 sequences of which 13,321 have gaps followed by the subset used for comparisons based on CYP3A4 harboring 4,775 sequences of which 1,899 have gaps and to the right the subdataset of OleP analogs with 306 sequences of which 45 have gaps.
- **Fig. 6**: shows the 3DM analysis of residue S240, which is assigned as core position 211 in the 3DM database for P450 monooxygenase analogs with detailed occurrence of each of the amino acids at this position. Here, three different sub-datasets are shown: the full dataset to the left covering 86,622 sequences of which 4,385 have gaps followed by subset used for comparisons based on CYP3A4 harboring 6,452 sequences of which 222 have gaps and to the right the subdataset of OleP analogs with 332 sequences of which 19 have gaps.
- **Fig. 7**: shows the 3DM analysis of residue V291, which is assigned as core position 281 in the 3DM database for P450 monooxygenase analogs with detailed occurrence of each of the amino acids at this position. Here three different sub-datasets are shown: the full dataset to the left covering 89,903 sequences of which 1,104 have gaps followed by subset used for comparisons based on CYP3A4 harboring 6,595 sequences of which 79 have gaps and to the right the subdataset of OleP analogs with 351 sequences of which 0 have gaps.
- **Fig. 8**: shows the ¹H-NMR spectrum of the isolated product identified as UDCA.
- **Fig. 9**: shows the 13C-NMR spectrum of the isolated product identified as UDCA.

### Cited Literature

- S Kulprecha, T Ueda, T Nihira, T Yoshida and H Taguchi, Appl. Environ. Microbiol. 1985, 1985, 338
- S. Grobe, A. Wszo 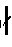 ek, H. Brundiek, M. Fekete, U. T. Bornscheuer, *Biotechnol. Lett.* 2020, 819
- R. K. Kuipers, H.-J. Joosten, W. J. H. van Berkel, N. G. H. Leferink, E. Rooijen, E. Ittmann, F. van Zimmeren, H. Jochens, U. Bornscheuer, G. Vriend et al., Proteins 2010, 78, 2101
- A. Li, C. G. Acevedo-Rocha, Z. Sun, T. Cox, J. L. Xu, M. T. Reetz, ChemBioChem 2018, 19, 221
- Berge et al. "Pharmaceutical Salts", J. Pharm. Sci. 66: 1-19 (1977)
- T. Omura, R. Sato, J. Biol. Chem. 1964, 239, 2370
- E. F. Pettersen, T. D. Goddard, C. C. Huang, G. S. Couch, D. M. Greenblatt, E. C. Meng, T. E. Ferrin, J. Comp. Chem. 2004, 25, 1605
- F. P. Guengerich, M. V. Martin, C. D. Sohl, Q. Cheng, Nature Prot. 2009, 4
- O. Gotoh, J. Biol. Chem. 1992, 1992, 81

## Claims

1. A process for 7-beta-hydroxylation of bile acid derivatives comprising:
(a) providing a bile acid derivative according to general formula (I) 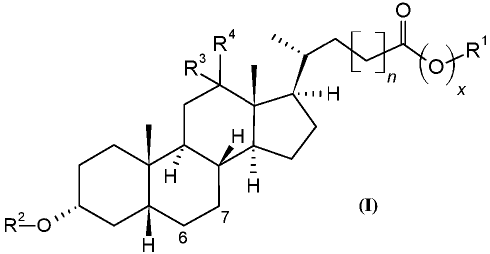 wherein
R¹ is selected from the group consisting of hydrogen atom, C1 to C30 alkyl group, C1 to C30 alkenyl group, C1 to C30 alkinyl group, C5 to C12 cycloalky group, C5 to C12 aryl group; and NR⁵R⁶ group, wherein R⁵ and R⁶ are independently selected from hydrogen atom, C1 to C10 alkyl group, C5 to C12 aryl group, -C(=O)-C1 to C10 alkyl group, - C(=O)-C5 to C12 aryl group and -NH-C1 to C5 alkyl-S(=O)₂-OH group;
R² is selected from the group consisting of hydrogen atom, -C(=O)-C1 to C30 alkyl group, -C(=O)-C5 to C12 aryl group -S(=O)₂-C1 to C30 alkyl group, -S(=O)₂-C5 to C12 aryl group, -S(=O)₂-OH group, salt of a - S(=O)₂-OH group, -P(=O)-OH-R⁷ group, salt of -P(=O)-OH-R⁷ group, wherein R⁷ is a hydrogen atom or a C1 to C5 alkyl group, -P(=O)(OH)₂ group, and a salt of -P(=O)(OH)₂ group;
R³, R⁴ are independently selected from hydrogen atom or -OR², wherein R² has the same meaning as above; or R³ and R⁴ together form =O;
n is zero or an integer in the range of from 1 to 5;
x is zero or 1;
(b) contacting the bile acid derivative according to general formula (I) with at least one cytochrome P450 monooxygenase analog, wherein the cytochrome P450 monooxygenase analog comprises an amino acid sequence according to SEQ ID NO: 1 with at least two mutations compared to the parent cytochrome P450 monooxygenase at positions selected from the group consisting of 84, 240, and 291 based on SEQ ID NO:1, wherein the parent cytochrome P450 monooxygenase of the cytochrome P450 monooxygenase analog is cytochrome P450 monooxygenase CYP107D1 (OleP) from *Streptomyces antibioticus* according to SEQ ID NO: 1;
thereby obtaining a bile acid derivative according to general formula (II) as a product, which has a beta-hydroxyl group in position 7, 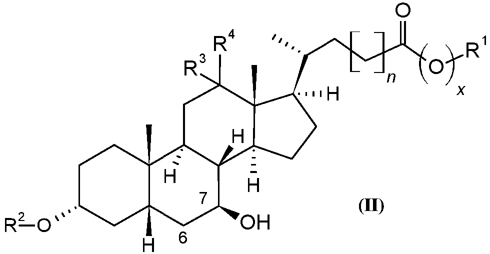
wherein R¹, R², R³, R⁴ and the indices n and x have the same meaning as for the bile acid derivative according to general formula (I).

2. The process of claim 1, wherein the bile acid derivative according to general formula (II) having a beta-hydroxyl group in position 7 is obtained with a bile acid derivative according to general formula (III) as a further product having a beta-hydroxyl group in position 6 (instead of position 7), 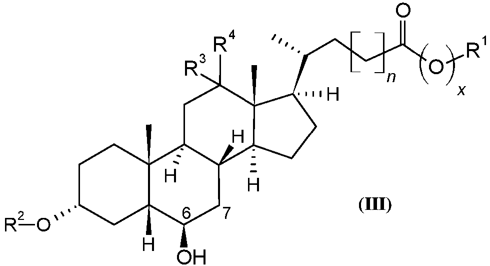
wherein R¹, R², R³, R⁴, R⁵ and the indices n and x have the same meaning as for the bile acid derivative according to general formula (I) in claim 1
wherein the molar ratio (II) : (III) is in the range of from 1:10 to 100:0.001, preferably in the range of from 4:10 to 100:0.001, more preferred in the range of from 1:1 to 100:0.001.

3. The process for 7-beta-hydroxylation of bile acid derivatives according to claim 1 or 2, comprising:
(a) providing a bile acid derivative according to general formula (la), 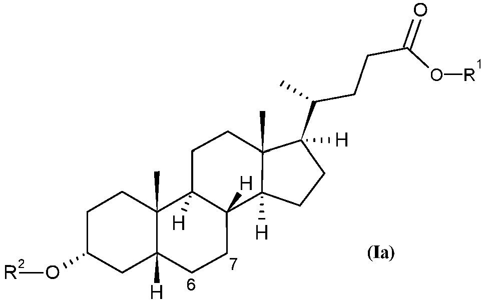 wherein
R¹ is selected from the group consisting of hydrogen atom, C1 to C30 alkyl group, C1 to C30 alkenyl group, C1 to C30 alkinyl group, C5 to C12 cycloalky group and C5 to C12 aryl group, preferably a hydrogen atom or a C1 to C30 alkyl group, more prefered a hydrogen atom;
R² is a hydrogen atom or a -C(=O)-C1- to C30 alkyl group, preferably a hydrogen atom;
(b) contacting the bile acid derivative according to general formula (Ia) with at least one cytochrome P450 monooxygenase analog, wherein the cytochrome P450 monooxygenase analog comprises at least two mutations compared to the parent cytochrome P450 monooxygenase;
thereby obtaining a bile acid derivative according to general formula (IIa) as a product, which has a beta-hydroxyl group in position 7, 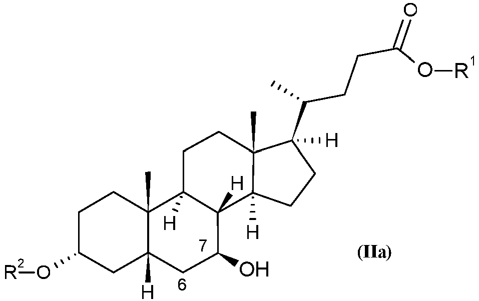
wherein R' and R² have the same meaning as for the bile acid derivative according to general formula (la).

4. The process of claim 3, wherein the bile acid derivative according to general formula (IIa) having a beta-hydroxyl group in position 7 is obtained with a bile acid derivative according to general formula (Illa) as a further product having a beta-hydroxyl group in position 6 (instead of position 7), 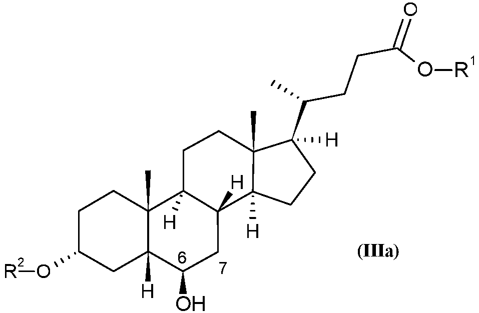
wherein R¹ and R² have the same meaning as for the bile acid derivative according to general formula (Ia) in claim 3,
wherein the molar ratio (IIa) : (Illa) is in the range of from 1:10 to 100:0.001, preferably in the range of from 4:10 to 100:0.001, more preferred in the range of from 1:1 to 100:0.001.

5. The process of any one of claims 1 to 4, wherein the bile acid derivative according to general formula (I) or (la) provided in (a) is lithocholic acid (LCA, R¹ = hydrogen atom, R², R³, R⁴ = hydrogen atom, n = 1, x = 1) and the bile acid derivative obtained by the process having general formula (II) or (IIa) is ursodeoxycholic acid (UDCA, each of R¹, R², R³, R⁴ = hydrogen atom, n = 1, x = 1).

6. The process of any one of claims 1 to 5, wherein the cytochrome P450 monooxygenase analog comprises an amino acid sequence according to SEQ ID NO: 1 with at least two mutations compared to the parent cytochrome P450 monooxygenase at positions selected from the group consisting of 84, 240, and 291 based on SEQ ID NO:1, wherein the at least two mutations comprised in the cytochrome P450 monooxygenase analog are that:
- position 84 is substituted with a non-aromatic amino acid, preferably with a non-aromatic amino acid selected from the group consisting of cysteine (Cys, C), glutamine (Gln, Q), methionine (Met, M), and alanine (Ala, A); and/or
- position 240 is substituted with alanine (Ala, A); and/or
- position 291 is substituted with an aliphatic or acidic amino acid, preferably with an amino acid selected from the group consisting of alanine (Ala, A), aspartic acid (Asp, D), and glycine (Gly, G).

7. The process of any one of claims 1 to 6, wherein the cytochrome P450 monooxygenase analog comprises at least a substitution at position 291 and at least one further substitution at position 84 and/or 240, wherein preferably the cytochrome P450 monooxygenase analog comprises mutations in that:
- position 291 is substituted with an aliphatic or acidic amino acid, preferably with an amino acid selected from the group consisting of alanine (Ala, A), aspartic acid (Asp, D), and glycine (Gly, G); and
- position 84 is substituted with a non-aromatic amino acid, preferably with a non-aromatic amino acid selected from the group consisting of cysteine (Cys, C), glutamine (Gln, Q), methionine (Met, M), and alanine (Ala, A); and/or
- position 240 is substituted with alanine (Ala, A).

8. The process of any one of claims 1 to 7, wherein the cytochrome P450 monooxygenase analog comprises or consists of an amino acid sequence selected from the group consisting of SEQ ID NO: 2 (F84Q/V291A), SEQ ID NO: 3 (S240A/V291A), SEQ ID NO: 4 (F84A/V291A), SEQ ID NO: 5 (F84C/S240A/V291D), SEQ ID NO: 6 (F84C/S240A/V291A), SEQ ID NO: 7 (F84C/S240A/V291G), SEQ ID NO: 8 (F84Q/S240A/V291A), SEQ ID NO: 9 (F84M/S240A/V291A), SEQ ID NO: 10 (F84Q/S240A/V291D), SEQ ID NO: 11 (F84A/S240A/V291A) and SEQ ID NO: 12 (F84Q/S240A/V291G), wherein the cytochrome P450 monooxygenase analog preferably comprises or consists of an amino acid sequence according to SEQ ID NO: 12 (F84Q/S240A/V291G).

9. The process of any one of claims 1 to 8, wherein the cytochrome P450 monooxygenase analog comprises one or more further amino acid(s) prior to the first amino acid of any one of SEQ ID NO: 2 to 12 and/or one or more further amino acid(s) after the last amino acid of any one of SEQ ID NO: 2 to 12, preferably one or more further amino acid(s) prior to the first amino acid of any one of SEQ ID NO: 2 to 12.

10. A cytochrome P450 monooxygenase analog, wherein the parent cytochrome P450 monooxygenase of the cytochrome P450 monooxygenase analog is cytochrome P450 monooxygenase CYP107D1 (OleP) from *Streptomyces antibioticus* according to SEQ ID NO: 1, wherein the cytochrome P450 monooxygenase analog comprises an amino acid sequence according to SEQ ID NO: 1 having at least two mutations compared to the parent cytochrome P450 monooxygenase at positions selected from the group consisting of 84, 240, and 291 based on SEQ ID NO:1,
wherein preferably the at least two mutations comprised in the cytochrome P450 monooxygenase analog are that:
- position 84 is substituted with a non-aromatic amino acid, preferably with a non-aromatic amino acid selected from the group consisting of cysteine (Cys, C), glutamine (Gln, Q), methionine (Met, M), and alanine (Ala, A); and/or
- position 240 is substituted with alanine (Ala, A); and/or
- position 291 is substituted with an aliphatic or acidic amino acid, preferably with an amino acid selected from the group consisting of alanine (Ala, A), aspartic acid (Asp, D), and glycine (Gly, G).

11. The cytochrome P450 monooxygenase analog of claim 10, wherein the cytochrome P450 monooxygenase analog comprises at least a substitution at position 291 and at least one further substitution at position 84 and/or 240,
wherein preferably the cytochrome P450 monooxygenase analog comprises mutations in that:
- position 291 is substituted with an aliphatic or acidic amino acid, preferably with an amino acid selected from the group consisting of alanine (Ala, A), aspartic acid (Asp, D), and glycine (Gly, G); and
- position 84 is substituted with a non-aromatic amino acid, preferably with a non-aromatic amino acid selected from the group consisting of cysteine (Cys, C), glutamine (Gln, Q), methionine (Met, M), and alanine (Ala, A); and/or
- position 240 is substituted with alanine (Ala, A).

12. The cytochrome P450 monooxygenase analog of claim 10 or 11, wherein the cytochrome P450 monooxygenase analog comprises or consists of an amino acid sequence selected from the group consisting of SEQ ID NO: 2 (F84Q/V291A), SEQ ID NO: 3 (S240A/V291A), SEQ ID NO: 4 (F84A/V291A), SEQ ID NO: 5 (F84C/S240A/V291D), SEQ ID NO: 6 (F84C/S240A/V291A), SEQ ID NO: 7 (F84C/S240A/V291G), SEQ ID NO: 8 (F84Q/S240A/V291A), SEQ ID NO: 9 (F84M/S240A/V291A), SEQ ID NO: 10 (F84Q/S240A/V291D), SEQ ID NO: 11 (F84A/S240A/V291A) and SEQ ID NO: 12 (F84Q/S240A/V291G), wherein the cytochrome P450 monooxygenase analog preferably comprises or consists of an amino acid sequence according to SEQ ID NO: 12 (F84Q/S240A/V291G).

13. The cytochrome P450 monooxygenase analog of any one of claims 10 to 12, wherein the cytochrome P450 monooxygenase analog comprises one or more further amino acid(s) prior to the first amino acid of any one of SEQ ID NO: 2 to 12 and/or one or more further amino acid(s) after the last amino acid of any one of SEQ ID NO: 2 to 12, preferably one or more further amino acid(s) prior to the first amino acid of any one of SEQ ID NO: 2 to 12.

14. A polynucleotide encoding the cytochrome P450 monooxygenase analog of any one of claims 10 to 13, preferably encoding the cytochrome P450 monooxygenase analog of SEQ ID NO: 12.

15. A vector comprising the polynucleotide encoding the cytochrome P450 monooxygenase analog of claim 14, wherein the vector is preferably an expression vector.

16. A host cell comprising the cytochrome P450 monooxygenase analog of any one of claims 10 to 13 and/or the polynucleotide of claim 14, and/or the vector of claim 15, wherein the host cell is preferably a bacteria cell, more preferred a bacteria cell belonging to the genus *Escherichia,* more preferred an *Escherichia coli* cell, more preferred an *Escherichia coli* cell selected from BL21 (DE3), BL21 (DE3) Gold, C41 (DE3) and C43 (DE3), more preferred *E. coli* C43 (DE3).

## Patentansprüche

1. Verfahren zur 7-beta-Hydroxylierung von Gallensäurederivaten, umfassend:
(a) Bereitstellen eines Gallensäurederivats gemäß der allgemeinen Formel (I) 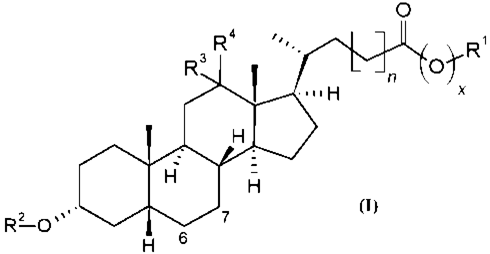 wobei
R¹ aus der Gruppe bestehend aus Wasserstoffatom, C1-bis C30-Alkylgruppe, C1- bis C30-Alkenylgruppe, C1- bis C30-Alkinylgruppe, C5- bis C12-Cycloalkylgruppe, C5- bis C12-Arylgruppe und NR⁵R⁶-Gruppe ausgewählt ist, wobei R⁵ und R⁶ unabhängig aus Wasserstoffatom, C1- bis C10-Alkylgruppe, C5- bis C12-Arylgruppe, -C(=O)-C1- bis -C10-Alkylgruppe, -C(=O)-C5- bis -C12-Arylgruppe und -NH-C1-bis -C5-Alkyl-S(=O)₂-OH-Gruppe ausgewählt sind;
R² aus der Gruppe bestehend aus Wasserstoffatom, - C(=O)-C1- bis -C30-Alkylgruppe, -C(=O)-C5- bis -C12-Arylgruppe, -S(=O)₂-C1- bis -C30-Alkylgruppe, -S(=O)₂-C5-bis -C12-Arylgruppe, -S(=O)₂-OH-Gruppe, Salz einer - S(=O)₂-OH-Gruppe, -P(=O)-OH-R⁷-Gruppe, Salz von -P(=O)-OH-R⁷-Gruppe, wobei R⁷ für ein Wasserstoffatom oder eine C1- bis C5-Alkylgruppe, -P(=O)(OH)₂ -Gruppe und ein Salz von -P(=O)(OH)₂-Gruppe steht, ausgewählt ist;
R³, R⁴ unabhängig aus Wasserstoffatom oder -OR² ausgewählt sind, wobei R² die gleiche Bedeutung wie oben hat; oder R³ und R⁴ zusammen =O bilden;
n für Null oder eine ganze Zahl im Bereich von 1 bis 5 steht;
x für Null oder 1 steht;
(b) Inkontaktbringen des Gallensäurederivats gemäß der allgemeinen Formel (I) mit mindestens einem Cytochrom-P450-Monooxygenase-Analog, wobei das Cytochrom-P450-Monooxygenase-Analog eine Aminosäuresequenz gemäß SEQ ID NO: 1 mit mindestens zwei Mutationen gegenüber der Stamm-Cytochrom-P450-Monooxygenase an Positionen, die aus der Gruppe bestehend aus 84, 240 und 291 bezogen auf SEQ ID NO:1 ausgewählt sind, umfasst, wobei es sich bei der Stamm-Cytochrom-P450-Monooxygenase des Cytochrom-P450-Monooxygenase-Analogs um Cytochrom-P450-Monooxygenase CYP107D1 (OleP) aus *Streptomyces antibioticus* gemäß SEQ ID NO: 1 handelt;
wodurch ein Gallensäurederivat gemäß der allgemeinen Formel (II) als Produkt erhalten wird, das eine Beta-Hydroxylgruppe in Position 7 aufweist, 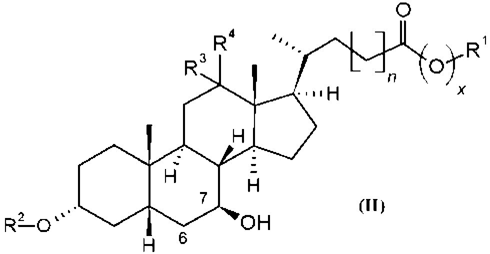
wobei R¹, R², R³, R⁴ und die Indizes n und x die gleiche Bedeutung wie für das Gallensäurederivat gemäß der allgemeinen Formel (I) haben.

2. Verfahren nach Anspruch 1, wobei das Gallensäurederivat gemäß der allgemeinen Formel (II) mit einer Beta-Hydroxylgruppe in Position 7 mit einem Gallensäurederivat gemäß der allgemeinen Formel (III) als weiteres Produkt mit einer Beta-Hydroxylgruppe in Position 6 (statt Position 7) erhalten wird, 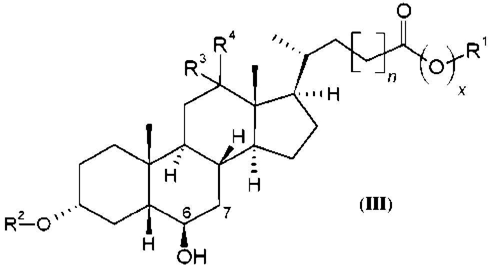
wobei R¹, R², R³, R⁴, R⁵ und die Indizes n und x die gleiche Bedeutung wie für das Gallensäurederivat gemäß der allgemeinen Formel (I) in Anspruch 1 haben,
wobei das Molverhältnis (II) : (III) im Bereich von 1:10 bis 100:0,001, vorzugsweise im Bereich von 4:10 bis 100:0,001, bevorzugter im Bereich von 1:1 bis 100:0,001 liegt.

3. Verfahren zur 7-beta-Hydroxylierung von Gallensäurederivaten gemäß Anspruch 1 oder 2, umfassend:
(a) Bereitstellen eines Gallensäurederivats gemäß der allgemeinen Formel (Ia), 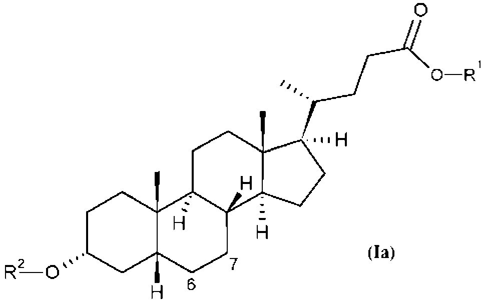 wobei
R¹ aus der Gruppe bestehend aus Wasserstoffatom, C1-bis C30-Alkylgruppe, C1- bis C30-Alkenylgruppe, C1- bis C30-Alkinylgruppe, C5- bis C12-Cycloalkylgruppe und C5-bis C12-Arylgruppe, vorzugsweise einem Wasserstoffatom oder einer C1- bis C30-Alkylgruppe, bevorzugter einem Wasserstoffatom ausgewählt ist;
R² für ein Wasserstoffatom oder eine -C(=O)-C1- bis - C30-Alkylgruppe, vorzugsweise ein Wasserstoffatom, steht;
(b) Inkontaktbringen des Gallensäurederivats gemäß der allgemeinen Formel (Ia) mit mindestens einem Cytochrom-P450-Monooxygenase-Analog, wobei das Cytochrom-P450-Monooxygenase-Analog mindestens zwei Mutationen gegenüber der Stamm-Cytochrom-P450-Monooxygenase umfasst;
wodurch ein Gallensäurederivat gemäß der allgemeinen Formel (IIa) als Produkt erhalten wird, das eine Beta-Hydroxylgruppe in Position 7 aufweist, 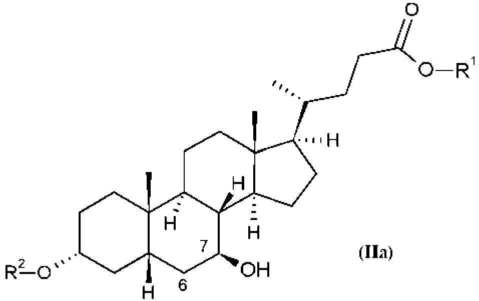
wobei R¹ und R² die gleiche Bedeutung wie für das Gallensäurederivat gemäß der allgemeinen Formel (Ia) haben.

4. Verfahren nach Anspruch 3, wobei das Gallensäurederivat gemäß der allgemeinen Formel (IIa) mit einer Beta-Hydroxylgruppe in Position 7 mit einem Gallensäurederivat gemäß der allgemeinen Formel (IIIa) als weiteres Produkt mit einer Beta-Hydroxylgruppe in Position 6 (statt Position 7) erhalten wird, 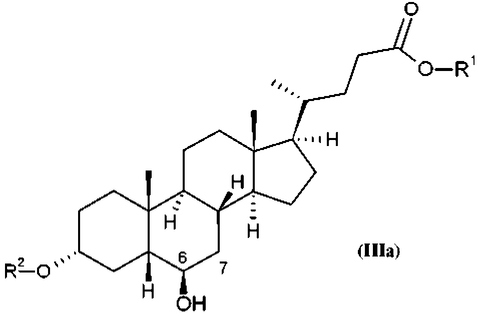
wobei R¹ und R² die gleiche Bedeutung wie für das Gallensäurederivat gemäß der allgemeinen Formel (Ia) in Anspruch 3 haben,
wobei das Molverhältnis (IIa) : (IIIa) im Bereich von 1:10 bis 100:0,001, vorzugsweise im Bereich von 4:10 bis 100:0,001, bevorzugter im Bereich von 1:1 bis 100:0,001 liegt.

5. Verfahren nach einem der Ansprüche 1 bis 4, wobei es sich bei dem in (a) bereitgestellten Gallensäurederivat gemäß der allgemeinen Formel (I) oder (Ia) um Lithocholsäure (LCA, R¹ = Wasserstoffatom, R², R³, R⁴ = Wasserstoffatom, n = 1, x = 1) und bei dem mit dem Verfahren erhaltenen Gallensäurederivat mit der allgemeinen Formel (II) oder (IIa) um Ursodesooxycholsäure (UDCA, R¹, R², R³, R⁴ jeweils = Wasserstoffatom, n = 1, x = 1) handelt.

6. Verfahren nach einem der Ansprüche 1 bis 5, wobei das Cytochrom-P450-Monooxygenase-Analog eine Aminosäuresequenz gemäß SEQ ID NO: 1 mit mindestens zwei Mutationen gegenüber der Stamm-Cytochrom-P450-Monooxygenase an Positionen, die aus der Gruppe bestehend aus 84, 240 und 291 bezogen auf SEQ ID NO:1 ausgewählt sind, umfasst, wobei die mindestens zwei in dem Cytochrom-P450-Monooxygenase-Analog enthaltenen Mutationen folgender Art sind:
- Position 84 ist durch eine nichtaromatische Aminosäure substituiert, vorzugsweise durch eine nichtaromatische Aminosäure, die aus der Gruppe bestehend aus Cystein (Cys, C), Glutamin (Gln, Q), Methionin (Met, M) und Alanin (Ala, A) ausgewählt ist; und/oder
- Position 240 ist durch Alanin (Ala, A) substituiert; und/oder
- Position 291 ist durch eine aliphatische oder saure Aminosäure substituiert, vorzugsweise durch eine Aminosäure, die aus der Gruppe bestehend aus Alanin (Ala, A), Asparaginsäure (Asp, D) und Glycin (Gly, G) ausgewählt ist.

7. Verfahren nach einem der Ansprüche 1 bis 6, wobei das Cytochrom-P450-Monooxygenase-Analog wenigstens eine Substitution an Position 291 und mindestens eine weitere Substitution an Position 84 und/oder 240 umfasst, wobei vorzugsweise das Cytochrom-P450-Monooxygenase-Analog Mutationen umfasst, indem:
- Position 291 durch eine aliphatische oder saure Aminosäure substituiert ist, vorzugsweise durch eine Aminosäure, die aus der Gruppe bestehend aus Alanin (Ala, A), Asparaginsäure (Asp, D) und Glycin (Gly, G) ausgewählt ist; und
- Position 84 durch eine nichtaromatische Aminosäure substituiert ist, vorzugsweise durch eine nichtaromatische Aminosäure, die aus der Gruppe bestehend aus Cystein (Cys, C), Glutamin (Gln, Q), Methionin (Met, M) und Alanin (Ala, A) ausgewählt ist; und/oder
- Position 240 durch Alanin (Ala, A) substituiert ist.

8. Verfahren nach einem der Ansprüche 1 bis 7, wobei das Cytochrom-P450-Monooxygenase-Analog eine Aminosäuresequenz umfasst oder daraus besteht, die aus der Gruppe bestehend aus SEQ ID NO: 2 (F84Q/V291A), SEQ ID NO: 3 (S240A/V291A), SEQ ID NO: 4 (F84A/V291A), SEQ ID NO: 5 (F84C/S240A/V291D), SEQ ID NO: 6 (F84C/S240A/V291A), SEQ ID NO: 7 (F84C/S240A/V291G), SEQ ID NO: 8 (F84Q/S240A/V291A), SEQ ID NO: 9 (F84M/S240A/V291A), SEQ ID NO: 10 (F84Q/S240A/V291D), SEQ ID NO: 11 (F84A/S240A/V291A) und SEQ ID NO: 12 (F84Q/S240A/V291G) ausgewählt ist, wobei das Cytochrom-P450-Monooxygenase-Analog vorzugsweise eine Aminosäuresequenz gemäß SEQ ID NO: 12 (F84Q/S240A/V291G) umfasst bzw. daraus besteht.

9. Verfahren nach einem der Ansprüche 1 bis 8, wobei das Cytochrom-P450-Monooxygenase-Analog eine oder mehrere weitere Aminosäure(n) vor der ersten Aminosäure einer von SEQ ID NO: 2 bis 12 und/oder eine oder mehrere weitere Aminosäure(n) nach der letzten Aminosäure einer von SEQ ID NO: 2 bis 12 umfasst, vorzugsweise eine oder mehrere weitere Aminosäure(n) vor der ersten Aminosäure einer von SEQ ID NO: 2 bis 12 umfasst.

10. Cytochrom-P450-Monooxygenase-Analog, wobei es sich bei der Stamm-Cytochrom-P450-Monooxygenase des Cytochrom-P450-Monooxygenase-Analogs um Cytochrom-P450-Monooxygenase CYP107D1 (OleP) aus *Streptomyces antibioticus* gemäß SEQ ID NO: 1 handelt, wobei das Cytochrom-P450-Monooxygenase-Analog eine Aminosäuresequenz gemäß SEQ ID NO: 1 mit mindestens zwei Mutationen gegenüber der Stamm-Cytochrom-P450-Monooxygenase an Positionen, die aus der Gruppe bestehend aus 84, 240 und 291 bezogen auf SEQ ID NO:1 ausgewählt sind, umfasst,
wobei vorzugsweise die mindestens zwei in dem Cytochrom-P450-Monooxygenase-Analog enthaltenen Mutationen folgender Art sind:
- Position 84 ist durch eine nichtaromatische Aminosäure substituiert, vorzugsweise durch eine nichtaromatische Aminosäure, die aus der Gruppe bestehend aus Cystein (Cys, C), Glutamin (Gln, Q), Methionin (Met, M) und Alanin (Ala, A) ausgewählt ist; und/oder
- Position 240 ist durch Alanin (Ala, A) substituiert; und/oder
- Position 291 ist durch eine aliphatische oder saure Aminosäure substituiert, vorzugsweise durch eine Aminosäure, die aus der Gruppe bestehend aus Alanin (Ala, A), Asparaginsäure (Asp, D) und Glycin (Gly, G) ausgewählt ist.

11. Cytochrom-P450-Monooxygenase-Analog nach Anspruch 10, wobei das Cytochrom-P450-Monooxygenase-Analog wenigstens eine Substitution an Position 291 und mindestens eine weitere Substitution an Position 84 und/oder 240 umfasst,
wobei vorzugsweise das Cytochrom-P450-Monooxygenase-Analog Mutationen umfasst, indem:
- Position 291 durch eine aliphatische oder saure Aminosäure substituiert ist, vorzugsweise durch eine Aminosäure, die aus der Gruppe bestehend aus Alanin (Ala, A), Asparaginsäure (Asp, D) und Glycin (Gly, G) ausgewählt ist; und
- Position 84 durch eine nichtaromatische Aminosäure substituiert ist, vorzugsweise durch eine nichtaromatische Aminosäure, die aus der Gruppe bestehend aus Cystein (Cys, C), Glutamin (Gln, Q), Methionin (Met, M) und Alanin (Ala, A) ausgewählt ist; und/oder
- Position 240 durch Alanin (Ala, A) substituiert ist.

12. Cytochrom-P450-Monooxygenase-Analog nach Anspruch 10 oder 11, wobei das Cytochrom-P450-Monooxygenase-Analog eine Aminosäuresequenz umfasst oder daraus besteht, die aus der Gruppe bestehend aus SEQ ID NO: 2 (F84Q/V291A), SEQ ID NO: 3 (S240A/V291A), SEQ ID NO: 4 (F84A/V291A), SEQ ID NO: 5 (F84C/S240A/V291D), SEQ ID NO: 6 (F84C/S240A/V291A), SEQ ID NO: 7 (F84C/S240A/V291G), SEQ ID NO: 8 (F84Q/S240A/V291A), SEQ ID NO: 9 (F84M/S240A/V291A), SEQ ID NO: 10 (F84Q/S240A/V291D), SEQ ID NO: 11 (F84A/S240A/V291A) und SEQ ID NO: 12 (F84Q/S240A/V291G) ausgewählt ist, wobei das Cytochrom-P450-Monooxygenase-Analog vorzugsweise eine Aminosäuresequenz gemäß SEQ ID NO: 12 (F84Q/S240A/V291G) umfasst bzw. daraus besteht.

13. Cytochrom-P450-Monooxygenase-Analog nach einem der Ansprüche 10 bis 12, wobei das Cytochrom-P450-Monooxygenase-Analog eine oder mehrere weitere Aminosäure(n) vor der ersten Aminosäure einer von SEQ ID NO: 2 bis 12 und/oder eine oder mehrere weitere Aminosäure(n) nach der letzten Aminosäure einer von SEQ ID NO: 2 bis 12 umfasst, vorzugsweise eine oder mehrere weitere Aminosäure(n) vor der ersten Aminosäure einer von SEQ ID NO: 2 bis 12 umfasst.

14. Polynukleotid, codierend das Cytochrom-P450-Monooxygenase-Analog nach einem der Ansprüche 10 bis 13, vorzugsweise codierend das Cytochrom-P450-Monooxygenase-Analog unter SEQ ID NO: 12.

15. Vektor, umfassend das Polynukleotid, das das Cytochrom-P450-Monooxygenase-Analog nach Anspruch 14 codiert, wobei es sich bei dem Vektor vorzugsweise um einen Expressionsvektor handelt.

16. Wirtszelle, umfassend das Cytochrom-P450-Monooxygenase-Analog nach einem der Ansprüche 10 bis 13 und/oder das Polynukleotid nach Anspruch 14 und/oder den Vektor nach Anspruch 15, wobei es sich bei der Wirtszelle vorzugsweise um eine Bakterienzelle, bevorzugter um eine Bakterienzelle, die zur Gattung *Escherichia* gehört, bevorzugter um eine *Escherichia* coli-Zelle, bevorzugter um eine *Escherichia* coli-Zelle, die aus BL21 (DE3), BL21 (DE3) Gold, C41 (DE3) und C43 (DE3) ausgewählt ist, bevorzugter um *E. coli* C43 (DE3) handelt.

## Revendications

1. Procédé de 7-bêta-hydroxylation de dérivés d'acide biliaire comprenant :
(a) la fourniture d'un dérivé d'acide biliaire selon la formule générale (I) 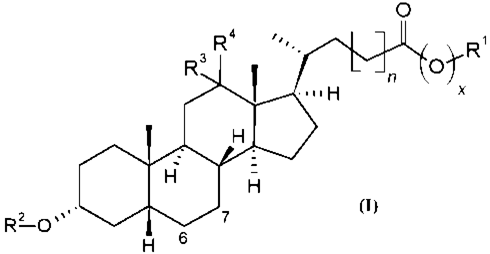 dans laquelle
R¹ est choisi dans le groupe constitué par un atome d'hydrogène, un groupe alkyle en C1 à C30, un groupe alcényle en C1 à C30, un groupe alcynyle en C1 à C30, un groupe cycloalkyle en C5 à C12, un groupe aryle en C5 à C12 ; et un groupe NR⁵R⁶, dans lequel R⁵ et R⁶ sont choisis indépendamment parmi un atome d'hydrogène, un groupe alkyle en C1 à C10, un groupe aryle en C5 à C12, un groupe -C(=O)-alkyle en C1 à C10, un groupe -C(=O)-aryle en C5 à C12 et un groupe -NH-alkyle en C1 à C5-S(=O)₂-OH ;
R² est choisi dans le groupe constitué par un atome d'hydrogène, un groupe -C(=O)-alkyle en C1 à C30, un groupe -S(=O)-aryle en C5 à C12, un groupe -S(=O)₂-alkyle en C1 à C30, un groupe -S(=O)₂-aryle en C5 à C12, un groupe -S(=O)₂-OH, un sel d'un groupe -S(=O)₂-OH, un groupe -P(=O)-OH-R⁷, un sel du groupe -P(=O)-OH-R⁷, dans lequel R⁷ est un atome d'hydrogène ou un groupe alkyle en C1 à C5, un groupe -P(=O) (OH)₂ et un sel du groupe - P(=O)(OH)₂ ;
R³, R⁴ sont choisis indépendamment parmi un atome d'hydrogène ou -OR², dans lequel R² a la même signification que ci-dessus ; ou R³ et R⁴ forment ensemble =O ;
n est zéro ou un entier dans la plage de 1 à 5 ;
x est zéro ou 1 ;
(b) la mise en contact du dérivé d'acide biliaire selon la formule générale (I) avec au moins un analogue de cytochrome P450 monooxygénase, l'analogue de cytochrome P450 monooxygénase comprenant une séquence d'acides aminés selon la SEQ ID NO: 1 ayant au moins deux mutations par rapport à la cytochrome P450 monooxygénase parente à des positions choisies dans le groupe constitué par 84, 240 et 291 sur la base de SEQ ID NO: 1, où la cytochrome P450 monooxygénase parente de l'analogue de cytochrome P450 monooxygénase est la cytochrome P450 monooxygénase CYP107D1 (OleP) de Streptomyces antibioticus selon la SEQ ID NO: 1 ;
obtenant ainsi un dérivé d'acide biliaire selon la formule générale (II) comme produit, qui possède un groupe bêta-hydroxyle en position 7, 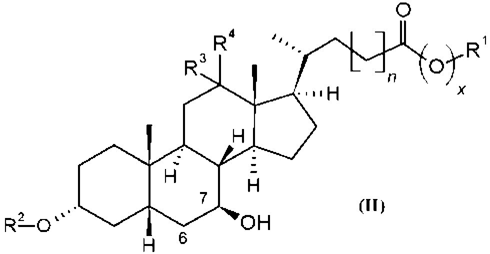
dans laquelle R¹, R², R³, R⁴ et les indices n et x ont la même signification que pour le dérivé d'acide biliaire selon la formule générale (I).

2. Procédé selon la revendication 1, dans lequel le dérivé d'acide biliaire selon la formule générale (II) ayant un groupe bêta-hydroxyle en position 7 est obtenu avec un dérivé d'acide biliaire selon la formule générale (III) en tant que produit supplémentaire ayant un groupe bêta-hydroxyle en position 6 (au lieu de la position 7), 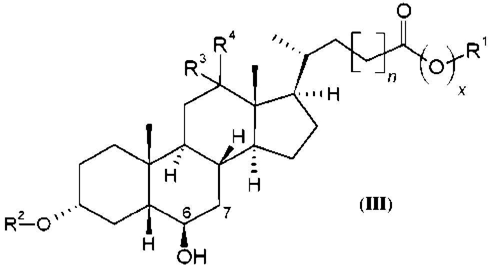
dans laquelle R¹, R², R³, R⁴, R⁵ et les indices n et x ont la même signification que pour le dérivé d'acide biliaire selon la formule générale (I) dans la revendication 1
dans lequel le rapport molaire (II) : (III) est dans la plage de 1 : 10 à 100 : 0,001, de préférence dans la plage de 4 : 10 à 100 : 0,001, plus préférablement dans la plage de 1 : 1 à 100 : 0,001.

3. Procédé de 7-bêta-hydroxylation de dérivés d'acide biliaire selon la revendication 1 ou 2, comprenant :
(a) la fourniture d'un dérivé d'acide biliaire selon la formule générale (Ia), 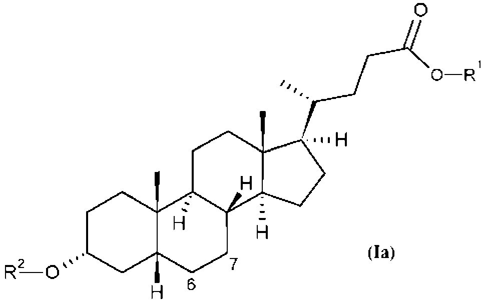 dans laquelle
R¹ est choisi dans le groupe constitué par un atome d'hydrogène, un groupe alkyle en C1 à C30, un groupe alcényle en C1 à C30, un groupe alcynyle en C1 à C30, un groupe cycloalkyle en C5 à C12 et un groupe aryle en C5 à C12, de préférence un atome d'hydrogène ou un groupe alkyle en C1 à C30, plus préférablement un atome d'hydrogène ;
R² est un atome d'hydrogène ou un groupe -C(=O)-alkyle en C1- à C30, de préférence un atome d'hydrogène ;
(b) la mise en contact du dérivé d'acide biliaire selon la formule générale (Ia) avec au moins un analogue de cytochrome P450 monooxygénase, l'analogue de cytochrome P450 monooxygénase comprenant au moins deux mutations par rapport à la cytochrome P450 monooxygénase parente ;
obtenant ainsi un dérivé d'acide biliaire selon la formule générale (IIa) comme produit, qui possède un groupe bêta-hydroxyle en position 7, 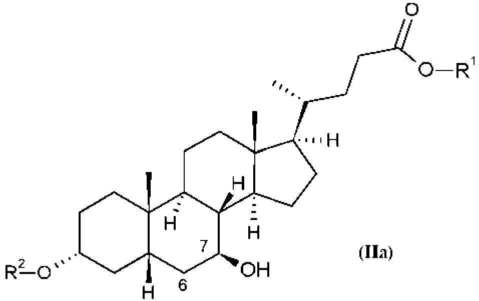
dans laquelle R¹ et R² ont la même signification que pour le dérivé d'acide biliaire selon la formule générale (Ia).

4. Procédé selon la revendication 3, dans lequel le dérivé d'acide biliaire selon la formule générale (IIa) ayant un groupe bêta-hydroxyle en position 7 est obtenu avec un dérivé d'acide biliaire selon la formule générale (IIIa) en tant qu'un produit supplémentaire ayant un groupe bêta-hydroxyle en position 6 (au lieu de la position 7), 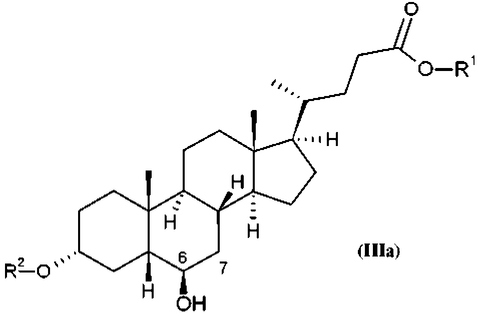
dans laquelle R¹ et R² ont la même signification que pour le dérivé d'acide biliaire selon la formule générale (Ia) dans la revendication 3,
dans lequel le rapport molaire (IIa) : (IIIa) est dans la plage de 1 : 10 à 100 : 0,001, préférablement dans la plage de 4 : 10 à 100 : 0,001, plus préférablement dans la plage de 1 : 1 à 100 : 0,001.

5. Procédé selon l'une quelconque des revendications 1 à 4, dans lequel le dérivé d'acide biliaire selon la formule générale (I) ou (Ia) fourni en (a) est l'acide lithocholique (LCA, R¹ = atome d'hydrogène, R², R³, R⁴ = atome d'hydrogène, n = 1, x = 1) et le dérivé d'acide biliaire obtenu par le procédé ayant la formule générale (II) ou (IIa) est l'acide ursodésoxycholique (UDCA, chacun parmi R¹, R², R³, R⁴ = atome d'hydrogène, n = 1, x = 1).

6. Procédé selon l'une quelconque des revendications 1 à 5, dans lequel l'analogue de cytochrome P450 monooxygénase comprend une séquence d'acides aminés selon la SEQ ID NO: 1 ayant au moins deux mutations par rapport à la cytochrome P450 monooxygénase parente aux positions choisies dans le groupe constitué par 84, 240 et 291 sur la base de SEQ ID NO: 1, où les au moins deux mutations comprises dans l'analogue de cytochrome P450 monooxygénase sont en ce que :
- la position 84 est substituée par un acide aminé non aromatique, de préférence par un acide aminé non aromatique choisi dans le groupe constitué par la cystéine (Cys, C), la glutamine (Gln, Q), la méthionine (Met, M), et l'alanine (Ala, A) ; et/ou
- la position 240 est substituée par l'alanine (Ala, A) ; et/ou
- la position 291 est substituée par un acide aminé aliphatique ou acide, de préférence par un acide aminé choisi dans le groupe constitué par l'alanine (Ala, A), l'acide aspartique (Asp, D) et la glycine (Gly, G).

7. Procédé selon l'une quelconque des revendications 1 à 6, dans lequel l'analogue de cytochrome P450 monooxygénase comprend au moins une substitution à la position 291 et au moins une substitution supplémentaire à la position 84 et/ou 240, où, de préférence, l'analogue de cytochrome P450 monooxygénase comprend des mutations en ce que :
- la position 291 est substituée par un acide aminé aliphatique ou acide, de préférence par un acide aminé choisi dans le groupe constitué par l'alanine (Ala, A), l'acide aspartique (Asp, D) et la glycine (Gly, g) ; et
- la position 84 est substituée par un acide aminé non aromatique, de préférence par un acide aminé non aromatique choisi dans le groupe constitué par la cystéine (Cys, C), la glutamine (Gln, Q), la méthionine (Met, M), et l'alanine (Ala, A) ; et/ou
- la position 240 est substituée par l'alanine (Ala, A).

8. Procédé selon l'une quelconque des revendications 1 à 7, dans lequel l'analogue de cytochrome P450 monooxygénase comprend ou est constitué par une séquence d'acides aminés choisie dans le groupe constitué par SEQ ID NO: 2 (F84Q/V291A), SEQ ID NO: 3 (S240A/V291A), SEQ ID NO: 4 (F84A/V291A), SEQ ID NO: 5 (F84C/S240A/V291D), SEQ ID NO: 6 (F84C/S240A/V291A), SEQ ID NO: 7 (F84C/S240A/V291G), SEQ ID NO: 8 (F84Q/S240A/V291A), SEQ ID NO: 9 (F84M/S240A/V291A), SEQ ID NO: 10 (F84Q/S240A/V291D), SEQ ID NO: 11 (F84A/S240A/V291A) et la SEQ ID NO: 12 (F84Q/S240A/V291G), dans lequel l'analogue de cytochrome P450 monooxygénase comprend ou est constitué par une séquence d'acides aminés selon la SEQ ID NO: 12 (F84Q/S240A/V291G).

9. Procédé selon l'une quelconque des revendications 1 à 8, dans lequel l'analogue de cytochrome P450 monooxygénase comprend un ou plusieurs autre(s) acide(s) aminé(s) avant le premier acide aminé de l'une quelconque parmi les SEQ ID NO: 2 à 12 et/ou un ou plusieurs autre(s) acide(s) aminé(s) après le dernier acide aminé de l'une quelconque parmi les SEQ ID NO: 2 à 12, de préférence un ou plusieurs autre(s) acide(s) aminé(s) avant le premier acide aminé de l'une quelconque parmi les SEQ ID NO: 2 à 12.

10. Analogue de cytochrome P450 monooxygénase, dans lequel la cytochrome P450 monooxygénase parente de l'analogue de cytochrome P450 monooxygénase est la cytochrome P450 monooxygénase CYP107D1 (OleP) provenant de Streptomyces antibioticus selon la SEQ ID NO: 1, dans lequel l'analogue de cytochrome P450 monooxygénase comprend une séquence d'acides aminés selon la SEQ ID NO: 1 ayant au moins deux mutations par rapport à la cytochrome P450 monooxygénase parente à des positions choisies dans le groupe constitué par 84, 240 et 291 sur la base de la SEQ ID NO: 1,
dans lequel, de préférence, les au moins deux mutations comprises dans l'analogue de cytochrome P450 monooxygénase sont en ce que :
- la position 84 est substituée par un acide aminé non aromatique, de préférence par un acide aminé non aromatique choisi dans le groupe constitué par la cystéine (Cys, C), la glutamine (Gln, Q), la méthionine (Met, M), et l'alanine (Ala, A) ; et/ou
- la position 240 est substituée par l'alanine (Ala, A) ; et/ou
- la position 291 est substituée par un acide aminé aliphatique ou acide, de préférence par un acide aminé choisi dans le groupe constitué par l'alanine (Ala, A), l'acide aspartique (Asp, D) et la glycine (Gly, G).

11. Analogue de cytochrome P450 monooxygénase selon la revendication 10, dans lequel l'analogue de cytochrome P450 monooxygénase comprend au moins une substitution en position 291 et au moins une substitution supplémentaire en position 84 et/ou 240,
dans lequel de préférence, l'analogue de cytochrome P450 monooxygénase comprend des mutations en ce que :
- la position 291 est substituée par un acide aminé aliphatique ou acide, de préférence par un acide aminé choisi dans le groupe constitué par l'alanine (Ala, A), l'acide aspartique (Asp, D) et la glycine (Gly, g) ; et
- la position 84 est substituée par un acide aminé non aromatique, de préférence par un acide aminé non aromatique choisi dans le groupe constitué par la cystéine (Cys, C), la glutamine (Gln, Q), la méthionine (Met, M), et l'alanine (Ala, A) ; et/ou
- la position 240 est substituée par l'alanine (Ala, A).

12. Analogue de cytochrome P450 monooxygénase selon la revendication 10 ou 11, l'analogue de cytochrome P450 monooxygénase comprenant ou étant constitué par une séquence d'acides aminés choisie dans le groupe constitué par la SEQ ID NO: 2 (F84Q/V291A), SEQ ID NO: 3 (S240A/V291A), SEQ ID NO: 4 (F84A/V291A), SEQ ID NO: 5 (F84C/S240A/V291D), SEQ ID NO: 6 (F84C/S240A/V291A), SEQ ID NO: 7 (F84C/S240A/V291G), SEQ ID NO: 8 (F84Q/S240A/V291A), SEQ ID NO: 9 (F84M/S240A/V291A), SEQ ID NO: 10 (F84Q/S240A/V291D), SEQ ID NO: 11 (F84A/S240A/V291A) et la SEQ ID NO: 12 (F84Q/S240A/V291G), dans lequel l'analogue de cytochrome P450 monooxygénase comprend ou est constitué par une séquence d'acides aminés selon la SEQ ID NO: 12 (F84Q/S240A/V291G).

13. Analogue de cytochrome P450 monooxygénase selon l'une quelconque des revendications 10 à 12, dans lequel l'analogue de cytochrome P450 monooxygénase comprend un ou plusieurs acide(s) aminé(s) supplémentaire (s) avant le premier acide aminé de l'une quelconque les SEQ ID NO: 2 à 12 et/ou un ou plusieurs autre(s) acide(s) aminé(s) après le dernier acide aminé de l'une quelconque parmi les SEQ ID NO: 2 à 12, de préférence un ou plusieurs autre(s) acide(s) aminé(s) avant le premier acide aminé de l'une quelconque parmi les SEQ ID NO: 2 à 12.

14. Polynucléotide codant pour l'analogue de cytochrome P450 monooxygénase selon l'une quelconque des revendications 10 à 13, de préférence codant pour l'analogue de cytochrome P450 monooxygénase de la SEQ ID NO: 12.

15. Vecteur comprenant le polynucléotide codant pour l'analogue de cytochrome P450 monooxygénase selon la revendication 14, dans lequel le vecteur est de préférence un vecteur d'expression.

16. Cellule hôte comprenant l'analogue de cytochrome P450 monooxygénase selon l'une quelconque des revendications 10 à 13 et/ou le polynucléotide selon la revendication 14, et/ou le vecteur selon la revendication 15, dans laquelle la cellule hôte est de préférence une cellule bactérienne, plus préférablement une cellule bactérienne appartenant au genre Escherichia, plus préférablement une cellule d'Escherichia coli, plus préférablement une cellule d'Escherichia coli choisie parmi BL21 (DE3), BL21 (DE3) Gold, C41 (DE3) et C43 (DE3), plus préférablement E. coli C43 (DE3).
